# EUROPÄISCHE PATENTSCHRIFT

(11) **EP 0 490 224 B1**
(45) Veröffentlichungstag und Bekanntmachung des Hinweises auf die Patenterteilung: **13.03.1996**
(21) Anmeldenummer: 91120720.7
(22) Anmeldetag: 03.12.1991
(51) Int. Cl.: C07D 491/048, A01N 43/90

(54) **Salicylaldehyd- und Salicylsäurederivate sowie deren Schwefelanaloge, Verfahren und Zwischenprodukte zu ihrer Herstellung**
Derivatives of salicylic aldehyde and salicylic acid, sulfphuranalogs thereof, process and intermediates for their preparation
Dérivés d'aldéhyde salicylique et d'acide salicylique et leurs analogues à base de soufre, procédé et intermédiaires pour leur préparation

(30) Priorität: 14.12.1990 DE 4039940; 10.08.1991 DE 4126935
(43) Veröffentlichungstag der Anmeldung: 17.06.1992
(73) Patentinhaber: BASF Aktiengesellschaft, D-67063 Ludwigshafen (DE)
(72) Erfinder: Eicken, Karl, Dr., W-6706 Wachenheim (DE); Rheinheimer, Joachim, Dr., W-6700 Ludwigshafen (DE); Vogelbacher, Uwe Josef, Dr., W-6700 Ludwigshafen (DE); Westphalen, Karl-Otto, Dr., W-6720 Speyer (DE); Gerber, Matthias, Dr., W-6704 Mutterstadt (DE); Walter, Helmut, Dr., W-6719 Obrigheim (DE)

(56) Entgegenhaltungen:
- EP-A- 0 223 406
- EP-A- 0 249 707
- JOURNAL OF HETEROCYCLIC CHEMISTRY. Bd. 18, Nr. 3, Mai 1981, PROVO US Seiten 581 - 585; S. FURUKAWA ET AL.: "Pyrimidinylalkylthiols, furo(2,3-d)pyrimidines and derivatives"

## Beschreibung

Die vorliegende Erfindung betrifft Salicylaldehyd- und Salicylsäurederivate und deren Schwefelanaloge der Formel I, 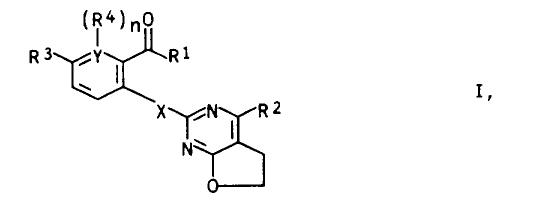 in der die Substituenten folgende Bedeutung haben:
- R¹: Wasserstoff
eine Succinylimidooxygruppe;
ein 5-gliedriger Heteroaromat, enthaltend ein bis drei Stickstoffatome, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
einen Rest -OR⁵, worin
R⁵
   Wasserstoff, ein Alkalimetallkation, das Äquivalent eines Erdalkalimetallkations oder ein organisches Ammoniumion;
eine C₃-C₁₂-Cycloalkylgruppe, welche ein bis drei C₁-C₄-Alkylreste tragen kann;
eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, C₃-C₁₂-Cycloalkyl, Phenyl, Phenoxy oder Phenylcarbonyl, wobei die aromatischen Reste ihrerseits ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein 5-gliedriger Heteroaromat, enthaltend ein bis drei Stickstoffatome oder ein Stickstoffatom und ein Sauerstoff- oder Schwefelatom, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
eine C₂-C₆-Alkylgruppe, welche in der 2-Position einen der folgenden Reste trägt: C₁-C₆-Alkoxyimino, C₃-C₆-Alkenyloxyimino, C₃-C₆-Halogenalkenyloxyimino oder Benzyloxyimino;
eine C₃-C₆-Alkenyl- oder eine C₃-C₆-Alkinylgruppe, wobei diese Gruppen ihrerseits ein bis fünf Halogenatome tragen können;
unsubstituiertes oder ein- bis dreifach durch C₁-C₄-Alkyl oder C₁-C₄-Alkoxy substituiertes oder ein- bis fünffach durch Halogen substituiertes Phenyl;
ein über ein Stickstoffatom gebundener 5-gliedriger aromatischer Heterocyclus mit ein bis vier Stickstoffatomen im Ring welcher ein bis drei der folgenden Substituenten tragen kann: Halogen, Nitro, Cyano, C₁-C₆-Alkoyl, C₁-C₆-Halogenalkyl, C₁-C₆-Alkoxy, C₁-C₆-Alkylthio;
eine Gruppe -N=CR⁶R⁷, worin R⁶ und R⁷ C₁-C₂₀-Alkyl, welches einen Phenylrest, eine C₁-C₄-Alkoxy- und/oder eine C₁-C₄-Alkylthiogruppe tragen kann, Phenyl oder gemeinsam eine C₃-C₁₂-Alkylenkette, welche ein bis drei C₁-C₃-Alkylgruppen tragen kann, bedeuten;
einen Rest 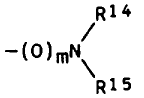
in dem m die Werte 0 und 1 annimmt und R¹⁴ und R¹⁵, die gleich oder unterschiedlich sein können, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino, C₃-C₆-Cycloalkyl, Phenyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
C₃-C₆-Cycloalkyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
Phenyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
R⁶ zusammen mit R⁷ eine 4- bis 7-gliedrige Alkylenkette, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio und in der eine CH2-Gruppe durch Sauerstoff, Schwefel oder -NH ersetzt sein kann;
eine Gruppe 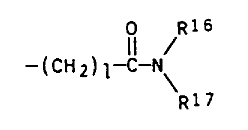
in der R¹⁶ und R¹⁷ für Wasserstoff, C₁-C₆-Alkyl, C₃-C₆-Alkenyl oder C₃-C₆-Alkinyl stehen und 1 die Werte 1, 2, 3 oder 4 bedeutet;
oder eine Gruppe

   -(CH₂)ₗ-S(=O)ₖ-R¹⁸
in der R¹⁸ für C₁-C₆-Alkyl, C₃-C₆-Alkenyl oder C₃-C₆-Alkinyl, l für 1, 2, 3 oder 4 und k für 0, 1 oder 2 stehen;
einen Rest OR⁵, worin R⁵ eine Gruppe 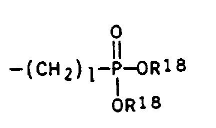
darstellt, in der R¹⁸ und l die obengenannte Bedeutung haben; oder einen Rest 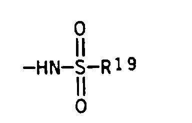
in dem R¹⁹ für die Reste C₁-C₆-Alkyl oder Phenyl steht, die ihrerseits ein bis vier der folgenden Substituenten tragen können; Halogen, Nitro, Cyano, C₁-C₆-Alkyl;
- R: C₁-C₄-Alkyl, C₁-C₄-Alkoxy, C₁-C₄-Halogenalkoxy oder Halogen;
- R³: Wasserstoff oder zusammen mit R⁴ eine Gruppe CH₂=CH-CH=CH₂ oder N=CH-CH=CH₂;
- R⁴: Wasserstoff, Halogen, C₁-C₄-Alkoxy, C₁-C₄-Halogenalkoxy, Aryloxy, C₁-C₄-Halogenalkyl, Formyl, C₁-C₈-Alkoxycarbonyl;
einen gegebenenfalls ein bis dreifach durch Halogen, Nitro, Cyano, C₁-C₆-Alkyl, C₁-C₆-Halogenalkyl, C₁-C₆-Alkoxy, C₁-C₆-Alkylthio substituierten, im Fall von Halogen als Substituent ein- bis fünffach durch Halogen, Nitro, Cyano, C₁-C₆-Alkyl, C₁-C₆-Halogenalkyl, C₁-C₆-Alkoxy, C₁-C₆-Alkylthio substituierten Phenylrest 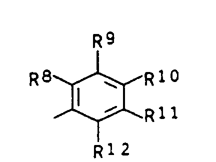
worin
R₈ - R₁₂
Wasserstoff, Halogen, Cyano, Nitro;
eine C₃-C₆-Alkenyl-, C₃-C₆-Alkenyloxy-, C₃-C₆-Alkinyloxy- oder eine C₃-C₆-Alkinylgruppe, wobei diese Gruppen ihrerseits ein bis fünf Halogenatome tragen können;
Di-C₁-C₄-alkylamino, C₃-C₈-Cycloalkyl, welches ein bis drei C₁-C₄-Alkylreste tragen kann;
C₁-C₁₀-Alkoxycarbonyl, C₁-C₄-Alkylthio;
eine Phenoxygruppe, wobei der aromatische Rest ein bis fünf Halogenatome und/oder ein bis drei der folgenden Reste tragen kann, C₁-C₄-Alkyl, C₁-C₄-Halogenalkyl, C₁-C₄-Halogenalkoxy, C₁-C₄-Alkoxy, C₁-C₄-Alkylthio;
eine C₁-C₁₀-Alkyl- oder Alkoxygruppe, welche ein bis fünf Halogenatome und/oder einen der folgenden Reste tragen kann: C₁-C₄-Alkoxy, C₁-C₄-Alkylthio, Phenyl oder Phenoxy, wobei die aromatischen Reste ihrerseits ein bis fünf Halogenatome und/oder ein bis drei der folgenden Reste tragen können: C₁-C₄-Alkyl, C₁-C₄-Halogenalkyl, C₁-C₄-Halogenalkoxy, C₁-C₄-Alkylthio;
bedeuten;
einen 5-gliedrigen Heteroaromaten, mit ein bis vier Stickstoffatomen oder ein bis zwei Stickstoffatomen sowie zusätzlich einem Schwefel- oder Sauerstoffatom im Ring, oder einen benzokondensierten 5-gliedrigen Heteroaromaten mit ein bis drei Stickstoffatomen oder einem Stickstoffatom und zusätzlich einem Sauerstoff- oder Schwefelatom im Ring, welche ein bis drei Halogenatome und/oder einen bis drei der folgenden Reste tragen können: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
einen Thienyl- oder Benzthienylrest, der ein bis drei Halogenatome und/oder einen bis drei der folgenden Reste tragen kann: C₁-C₄-Alkyl, C₁-C₂-Halogenalkyl oder Nitro;
einen Naphthyl- oder Chinolinylrest, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
oder Sauerstoff, wenn Y für Stickstoff und n für 1 steht;
- n: 1, wenn Y Kohlenstoff bedeutet oder wenn Y Stickstoff und R⁴ Sauerstoff bedeuten und 0, wenn Y für Stickstoff steht und R⁴ nicht Sauerstoff bedeutet;
- X: Sauerstoff oder Schwefel;
- Y: Stickstoff, wenn R³ Wasserstoff und n 0 bedeuten oder wenn R⁴ für Sauerstoff steht und n 1 bedeutet; Kohlenstoff, wenn R³ und R⁴ gemeinsam für eine Gruppe CH₂=CH-CH=CH₂ oder N=CH-CH=CH₂ stehen oder Kohlenstoff, wenn R³ Wasserstoff und n 1 bedeuten;
sowie umweltverträgliche Salze der Verbindungen I.

Weiterhin betrifft die Erfindung Verfahren zur Herstellung der Verbindung I sowie ihre Verwendung als Herbizide und Wachstumsregulatoren sowie neue Sulfone der Formel III 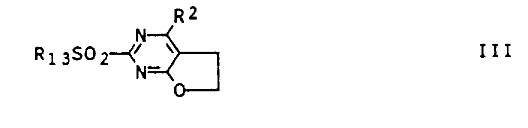 als Zwischenprodukte zur Herstellung der Verbindungen der Formel I. In der Formel III haben die Reste R und R¹³ die folgende Bedeutung:
- R: C₁-C₄-Alkyl, C₁-C₄-Alkoxy, C₁-C₄-Halogenalkoxy oder Halogen
- R¹³: C₁-C₁₂-Alkyl, gegebenenfalls durch Alkyl, Halogen oder Alkoxy substituiertes Phenyl.

In der Literatur (EP-A 223 406, EP-A 249 708, EP-A 287 072, EP-A 287 079 und EP-A 426 476) sind herbizid wirksame substituierte Salicylsäuren und deren Schwefelanaloge beschrieben. Ihre Wirkung ist jedoch unbefriedigend.

Aufgabe der vorliegenden Erfindung waren daher neue Salicylsäurederivate bzw. deren Schwefelanaloge mit verbesserten herbiziden Eigenschaften sowie mit pflanzenwachstumsregulierenden oder anderen im Pflanzenschutz nutzbaren Eigenschaften.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Salicylsäurederivate der vorstehend definierten allgemeinen Formel I ausgezeichnete pflanzenwachstumsregulierende Eigenschaften besitzen und daneben gute fungizide und nitrifikationshemmende Eigenschaften aufweisen. Als 5 Zwischenprodukte zur Herstellung der Verbindungen I wurden die neuen Sulfone III gefunden.

Verbindungen der Formel I erhält man beispielsweise, indem man ein entsprechend substituiertes Salicylsäurederivat der Formel II mit einer entsprechenden Verbindung der Formel III in Gegenwart einer Base umsetzt. 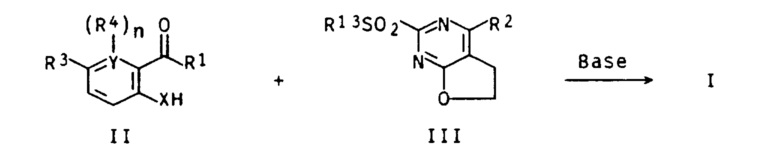 R¹³SO₂ in Formel III bedeutet eine übliche nucleofuge Abgangsgruppe, beispielsweise Arylsulfonyl wie Phenyl- oder substituiertes Phenylsulfonyl, wobei als Substituenten ein oder mehrere, z.B. 1 bis 3 niedermolekulare Alkyl- oder Alkoxyreste wie C₁-C₄-Alkyl oder Alkoxy oder Halogen, z.B. Chlor, Fluor oder Brom in Betracht kommen; oder Alkylsulfonyl wie C₁-C₄-Alkylsulfonyl, z.B. Methylsulfonyl. Als Base können Alkali- oder Erdalkalimetallhydride wie NaH und CaH₂, Alkalimetallhydroxide wie NaOH und KOH, Alkalimetallalkoholate wie Kalium-tert.-butylat, Alkalimetallcarbonate wie Na₂CO₃ und K₂CO₃, Alkalimetallamide wie NaNH₂ und Lithiumdiisopropylamid oder tertiäre Amine Verwendung finden. Bei Einsatz einer anorganischen Base kann man einen Phasentransferkatalysator zusetzen, wenn dies den Umsatz fördert.

Die Sulfone der allgemeinen Formel III erhält man indem man ein entsprechendes 2-Alkylthio- oder 2-Phenylthio-5,6-dihydrofuran[2,3-d]pyrimidin (s. Collect. Czech. Chem. Commun. 32, 1582 (1967)) 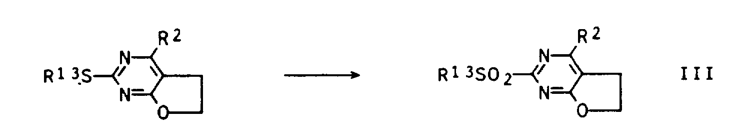 durch Oxidationsmittel wie z.B. Chlor in Wasser oder Wasserstoffperoxid in Eisessig oxydiert unter milden Bedingungen.

Die Zwischenprodukte der Formel II sind in Einzelfällen bekannt oder können nach üblichen Methoden, ausgehend von bekannten Vorprodukten hergestellt werden (vgl. z.B. EP-A-249 707, EP-A-315 889 oder JP-OS 2 056 469, Anm. Nr. 310741 v. 08.12.87). Folgende Zwischenprodukte der Formel II sind Gegenstand der älteren deutschen Anmeldung P 39 19 435.3 vom 14.06.89 und lassen sich, wenn X für ein Sauerstoffatom und A für einen über ein Kohlenstoffatom gebundenen Aromaten bzw. Heteroaromaten steht, gemäß dem untenstehenden Schema aus einer 1,3-Dicarbonylverbindung IV (mit R⁵ = gegebenenfalls phenylsubstituiertes C₁-C₁₀-Alkyl, insbesondere C₁-C₄-Alkyl) und einem α,β-ungesättigten Keton V aufbauen:

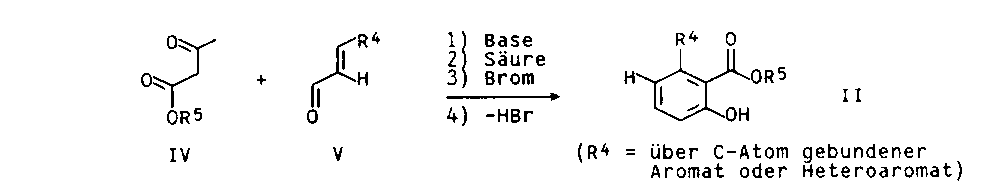

Alternativ dazu lassen sich die Zwischenprodukte der Formel II auch nach untenstehendem Schema aus einem Methylenphosphoran IV' (Ph = Phenyl) und einem α,β-ungesättigten Keton V herstellen:

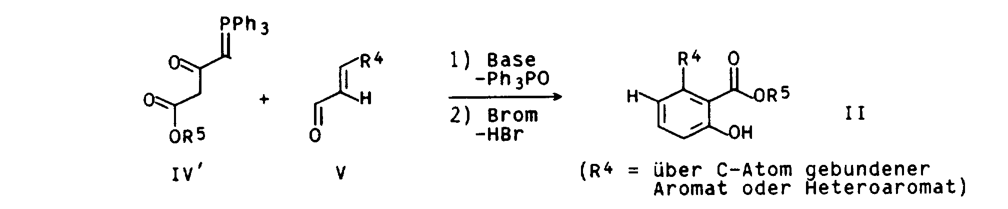

Dabei sind die Verbindungen IV, IV' und V im allgemeinen bekannt oder nach den üblichen Verfahren leicht herstellbar. Als Base kommen die oben genannten Verbindungen in Frage. Als Säure eignen sich starke Säuren, beispielsweise Chlorwasserstoff, Bromwasserstoff, Tetrafluoroborsäure, Toluolsulfonsäure oder Trifluoressigsäure. Die Bromwasserstoffabspaltung kann thermisch oder in Gegenwart einer Base, beisielsweise eines organischen Amins, durchgeführt werden.

Steht R⁴ in Formel II für einen über ein Stickstoffatom gebundenen Heteroaromaten und X für ein Sauerstoffatom, so kann man dieses Zwischenprodukt gemäß dem folgenden Schema aufbauen:

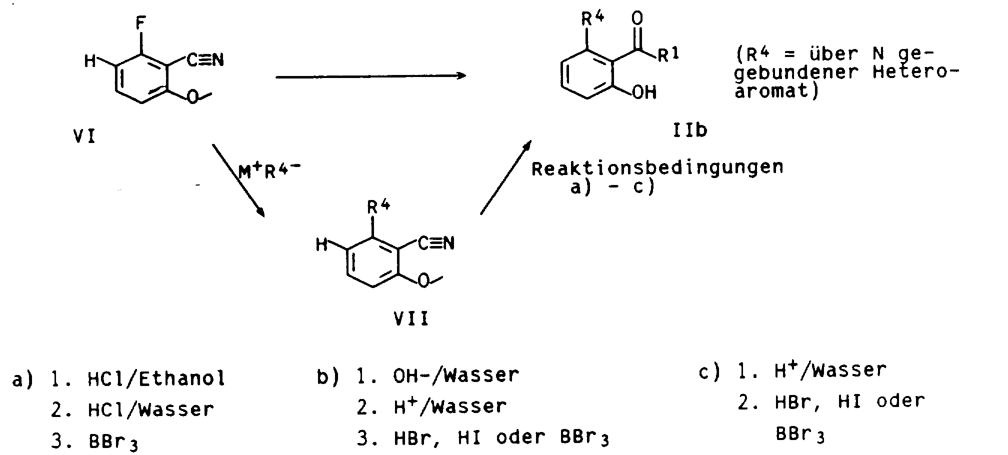

Dabei steht M⁺R⁴⁻ für das jeweilige Alkalimetallazolid. Als Alkohole kommen bei der Spaltung des Nitrils VII nach Variante a) insbesondere C₁-C₄-Alkylalkohole in Frage.

Die wie oben beschriebenen hergestellten Zwischenprodukte der Formel II fallen üblicherweise als Alkylester an. Diese lassen sich nach den bekannten Verfahren zu den Carbonsäuren hydrolysieren. Man kann letztere nun nach literaturbekannten Verfahren in verschiedene Ester überführen, die man zur Darstellung von Wirkstoffen der Formel I gemäß Anspruch 1 benötigt.

Wahlweise kann man auch die Zwischenprodukte der Formel VII nach allgemein bekannten Methoden mit Alkali- oder Tetraalkylammonium-hydroxiden zu den entsprechenden Amiden und dann mit Mineralsäuren, z.B. konz. Salzsäure, zu den Carbonsäuren und anschließend mit konz. Bromwasserstoffsäure zu den Salicylsäuren IIb umsetzen. Diese Schritte können bei Bedarf ohne Isolierung der Zwischenstufen durchgeführt werden.

Soweit es sich bei den in der beschriebenen Weise hergestellten Verbindungen der Formel I um Carbonsäuren handelt (wenn also R¹ Hydroxyl bedeutet), können hieraus andere beschriebene Verbindungen z.B. auch dadurch hergestellt werden, daß man die Carbonsäure zunächst auf übliche Weise in eine aktivierte Form wie ein Halogenid oder Imidazolid überführt und dieses dann mit der entsprechenden Hydroxylverbindung umsetzt. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Im Hinblick auf die Wirksamkeit sind Verbindungen I bevorzugt, in denen die Substituenten folgende Bedeutung haben:
- R¹: Wasserstoff, Succinylimidooxy,
5-gliedriges Heteroaryl wie Pyrrolyl, Pyrazolyl, Imidazolyl und Triazolyl, insbesondere Imidazolyl und Pyrazolyl, wobei der aromatische Rest über Stickstoff gebunden ist und seinerseits ein bis vier Halogenatome wie vorstehend genannt, insbesondere Fluor und Chlor und/oder ein bis zwei der folgenden Reste tragen kann:
Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
Alkoxy wie vorstehend genannt, mit ein bis vier Kohlenstoffatomen, Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
ein Rest OR⁵, worin
   R⁵ Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion;
   Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, welches ein bis fünf der vorstehend genannten Halogenatome, insbesondere Fluor und Chlor und/oder einen der folgenden Reste tragen kann:
   Cyano, Alkoxy bzw. Alkylthio mit ein bis vier Kohlenstoffatomen wie vorstehend genannt, insbesondere Methoxy, Ethoxy, 1-Methylethoxy und Methylthio;
   Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
   Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl.
   Phenyl, Phenoxy oder Phenylcarbonyl, wobei diese aromatischen Reste ihrerseits ein bis fünf Halogenatome wie vorstehend genannt, insbesondere Fluor, Chlor und Brom und/oder ein bis drei der folgenden Reste tragen können:
      Alkyl, Halogenalkyl, Alkoxy, Halogenalkoxy und/oder Alkylthio mit jeweils ein bis vier Kohlenstoffatomen wie im allgemeinen und im besonderen vorstehend genannt, oder
   5-gliedriges Hetaryl mit ein bis vier Stickstoffatomen im Ring, das über ein Stickstoffatom gebunden ist und ein bis vier der unter ggf. substituiert genannten Reste tragen kann. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 4-Iod-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl;
   C₁-C₁₀-Alkyl wie vorstehend genannt, welches ein bis fünf Halogenatome wie Fluor, Chlor, Brom oder Iod, insbesondere Fluor oder Chlor tragen kann und zusätzlich einen der folgenden Reste trägt:
   5-gliedriges Heteroaryl mit ein bis drei Stickstoffatomen wie vorstehend für R¹ genannt, wobei der aromatische Ring über Stickstoff oder Kohlenstoff gebunden sein kann, oder 5-gliedriges Heteroaryl enthaltend ein Stickstoffatom und ein Sauerstoff- oder Schwefelatom, das als Substituenten Alkyl, Halogenalkyl, Alkoxy oder Alkylthio wie unter R¹ genannt trägt.
   C₂-C₆-Alkyl, insbesondere C₂-C₄-Alkyl, das in 2-Stellung substituiert ist durch C₁-C₆-Alkoxyimino, z.B. Methoxy-, Ethoxy- oder Propoxyimino; C₃-C₆-Alkenyloxyimino wie 2-Propenyloxyimino, 2-Butenyloxyimino, 3-Butenyloxyimino; C₃-C₆-Halogenalkenyloxyimino wie 3,3-Dichlor-2-propenyloxyimino, 2,3,3-Trichlor-2propenoxyimino oder Benzyloxyimino.
   Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl, wobei diese Alkenyl- und Alkinylgruppen ein bis fünf der vorstehend im allgemeinen und im besonderen genannten Halogenatome tragen können, bedeutet;
   C₃-C₁₂-Cycloalkyl, insbesondere C₃-C₆-Cycloalkyl wie Cyclopropyl, Cyclobutyl, Cyclopentyl, Cyclohexyl, das gegebenenfalls durch ein bis drei C₁-C₄-Alkylreste substituiert ist;
   Phenyl, Phenyl ein bis dreifach substituiert durch C₁-C₄-Alkyl oder- Alkoxy wie Methyl, Ethyl, Propyl, Butyl, Methoxy, Ethoxy oder Phenyl substituiert durch ein bis fünf Halogenatome, z.B. Chlor oder Fluor;
ein Rest -N=CR⁶R⁷, worin
   R⁶ R⁷ unverzweigtes oder verzweigtes C₁-C₂₀-Alkyl, vorzugsweise C₁-C₁₅-Alkyl, insbesondere C₁-C₉-Alkyl, welches einen Phenyl, einen C₁-C₄-Alkoxy oder einen C₁-C₄-Alkylthiorest tragen kann; Phenyl oder gemeinsam C₃-C₁₂-Alkylen, vorzugsweise C₄-C₇-Alkylen, welches ein bis drei C₁-C₃-Alkylgruppen, vorzugsweise Methyl- oder Ethylgruppen tragen kann, bedeutet;
ein Rest 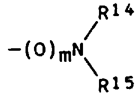 worin m die Werte 0 oder 1 annimmt und R¹⁴ und R¹⁵ im allgemeinen und im besonderen die für R⁵ genannten Alkyl-, Alkenyl-, Alkinyl-, Cycloalkyl- oder Phenylgruppen darstellen, die durch Halogen wie Fluor, Chlor oder Brom, oder durch Alkoxy und Alkylthio wie für R¹ genannt, oder durch Alkylcarbonyl oder Alkoxycarbonyl wie für R⁵ genannt substituiert sein können; beispielsweise seien für die Gruppierung -NR¹⁴R¹⁵ folgende Reste genannt: Dimethylamino, tert-Butylamino, Cyclohexylamino, 1-Cyano-1-cyclohexylamino, Isopropylamino, sek.-Butylamino, Methylamino, Diethylamino, Pyrrolidinyl, 1-Piperidyl, 4-Morpholinyl, Phenylamino, Methyl-phenyl-amino;
R⁶ zusammen mit R⁷ eine vier- bis siebengliedrige, insbesondere vier- bis sechsgliedrige Alkylenkette, die gegebenenfalls durch einen oder mehrere der unter "gegebenenfalls substituiert" genannten Reste substituiert ist und in der eine CH₂-Gruppe durch Sauerstoff, Schwefel oder NH ersetzt sein kann;
eine Gruppe 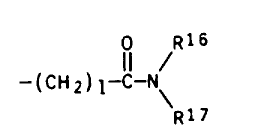 darstellt, in der l die Werte 1 bis 4, bevorzugt 1 und 2, annehmen kann, und R¹⁶ und R¹⁷ im allgemeinen und im speziellen Wasserstoff oder eine der unter R⁵ genannten Alkyl-, Alkenyl- und Alkinylgruppen bedeuten, bevorzugt Methyl und Ethyl;
oder eine Gruppe 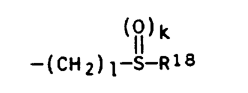 bildet, in der l die Werte 1 bis 4, bevorzugt 1 und 2, annehmen kann, und R¹⁸ im allgemeinen und im speziellen eine der unter R⁵ genannten Alkyl-, Alkenyl- und Alkinylgruppen bedeutet, bevorzugt Methyl und Ethyl;
ein Rest OR⁵, worin R⁵ eine Gruppierung 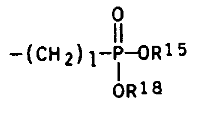 darstellt, in der l und R¹⁸ die oben angegebene Bedeutung haben;
ein Rest 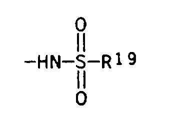 in dem R¹⁹ Alkyl oder ggf. substituiertes Phenyl darstellt, besonders bevorzugt sind Methyl, Phenyl und 4-Methylphenyl;
- R: im allgemeinen und im besonderen die bei R¹ genannten Alkylgruppen, Halogenalkylgruppen, Alkoxygruppen, Halogenalkoxygruppen und/oder mit jeweils ein bis vier Kohlenstoffatomen sowie Halogen wie Fluor, Chlor, Brom oder Iod, insbesondere Methyl, Chlor, Methoxy;
- R³: Wasserstoff oder zusammen mit R⁴ eine Gruppe CH₂=CH-CH=CH₂ oder N=CH-CH=CH₂;
- R⁴: Wasserstoff; Halogen wie bei R¹ genannt, insbesondere Fluor, Chlor und Brom;
C₁-C₄-Alkoxy wie Methoxy, Ethoxy, n-Propoxy, i-Propoxy, n-Butoxy, i-Butoxy, tert.-Butoxy;
C₁-C₃-Halogenalkoxy, z.B. C₁-C₃-Alkoxy substituiert durch 1 bis 5 Halogenatome wie Fluor, Chlor oder Brom, z.B. Trifluormethoxy, Difluormethoxy;
Aryloxy wie Phenoxy, gegebenenfalls substituiert durch Methyl, Fluor, Chlor oder Methoxy;
C₁-C₄-Halogenalkyl wie bei R¹ genannt, insbesondere C₁-C₂-Halogenalkyl wie Trifluormethyl;
Formyl;
C₂-C₈-Alkoxycarbonyl wie vorstehend für R¹ im einzelnen aufgeführt, insbesondere Methoxycarbonyl, Äthoxycarbonyl;
unsubstituiertes oder substituiertes Phenyl, worin als Substituenten R⁸ bis R¹ in Betracht kommen: Halogen wie Fluor, Chlor, Brom oder Iod; Cyano; Nitro, ggf. halogensubstituiertes Alkenyl, Alkenyloxy, Alkinyloxy oder Alkinyl mit jeweils 3 bis 6 Kohlenstoffatomen; Di-C₁-C₄-alkylamino wie Di-Methylamino, Di-Ethylamino, Di-Propylamino, Di-1-Methylethylamino, Di-Butylamino, Di-1-Methylpropylamino, Di-2-Methylpropylamino, Di-1,1-Dimethylethylamino, Ethylmethylamino, Propylmethylamino, 1-Methylethylmethylamino oder Butylmethylamino; gegebenenfalls alkylsubstituiertes Cycloalkyl wie vorstehend für R⁵ aufgeführt, Alkoxycarbonyl oder Alkylthio wie vorstehend für R⁵ aufgeführt, gegebenenfalls substituiertes Phenoxy wie unter R⁵ aufgeführt, C₁-C₁₀-Alkyl- oder Alkoxy, insbesondere C₁-C₆, bevorzugt C₁-C₄-Alkyl oder Alkoxy welche unsubstituiert oder durch die genannten Reste substituiert sind; beispielsweise seien die folgenden substituierten Phenylreste für R⁴ genannt:
2-Fluorphenyl, 3-Fluorphenyl, 4-Fluorphenyl, 2,6-Difluorphenyl, 2,4-Diflurophenyl, 2-Fluor-4-trifluormethylphenyl, 2,3-Difluorphenyl, 2-Chlorphenyl, 3-Chlorphenyl, 2-Iodphenyl, 2-Bromphenyl, 2-Chlor-6-fluorphenyl, Pentafluorphenyl, Pentachlorphenyl, 2,4-Dichlorphenyl, 2,6-Dichlorphenyl, 2-Chlor-4-fluorphenyl, 3,5-Dichlorphenyl, 2-Chlor-6-methylphenyl, 2,3,5-Trichlorphenyl, 2-Methylphenyl, 3-Methylphenyl, 4-Methylphenyl, 2,6-Dimethylphenyl, 2,4-Dimethylphenyl, 3,5-Dimethylphenyl, 2,4,6-Trimethylphenyl, 2-Chlor-4-methylphenyl, 2-Methoxyphenyl, 4-Methoxyphenyl, 4-Chlor-2-methoxyphenyl, 2-Trifluormethylphenyl, 2,3-Dimethyl-4-methoxyphenyl, 4-Dimethylamino-2-methylphenyl, 3-Cyanophenyl, 3-Nitrophenyl, 3-Phenoxyphenyl, 3-(3-Trifluormethylphenoxy)phenyl, 3-Trifluormethylphenyl,
gegebenenfalls substituiertes 5-gliedriges Hetaryl oder benzoanneliertes Hetaryl mit 1 bis 4 Stickstoffatomen wie für R¹ genannt oder ein bis 2 Stickstoffatomen und zusätzlich einem Schwefel- oder Sauerstoffatom wie gegebenenfalls substituiertes 5-gliedriges Hetaryl mit 2 bis 4 Stickstoffatomen wie für R¹ genannt oder ein bis 2 Stickstoffatomen und zusätzlich einem Schwefel- oder Sauerstoffatom wie Isoxazolyl, Oxazolyl, Thiazolyl, Thiadiazolyl.

Beispielsweise seien folgende Hetarylreste genannt:

Pyrrol-1-yl, 1-Methyl-pyrrol-2-yl, 1-Methyl-pyrrol-3-yl, Pyrazol-1-yl, 4-Methylpyrazol-1-yl, 3,5-Dimethylpyrazol-1-yl, 3,4,5-Trimethylpyrazol-1-yl, 4-Chlorpyrazol-1-yl, 4-Phenylpyrazol-1-yl, 4-Isopropylpyrazol-1-yl, 4-Nitropyrazol-1-yl, Imidazol-1-yl, 4,5-Dimethylimidazolyl, 2-Methyl-4,5-dichlorimidazolyl, 4(5)-Nitroimidazol-1-yl, [1,2,4]-Triazol-1-yl, 3(5)-Methyl-[1,2,4]-triazol-1-yl, [1,2,3]-Triazol-1-yl, 4,5-Dimethyl-[1,2,3]-triazol-1-yl, [1,2,3,4]-Tetrazol-1-yl, 1-Methylpyrazol-4-yl, 1-Phenylpyrazol-4-yl, 1,3,5-Trimethylpyrazol-4-yl, 1-Methylpyrazol-5-yl, 1-Phenylpyrazol-5-yl, 1-Methylpyrazol-3-yl, 1-Phenylpyrazol-3-yl, 1-Methylimidazol-2-yl, 1-Methylimidazol-5-yl, 1-Phenylimidazol-5-yl, 1-Phenyl-[1,2,3]-triazol-4-yl, Isoxazol-5-yl, Isoxazol-4-yl, 3-Methyl-isoxazol-5-yl, 3-Isopropyl-isoxazol-5-yl, 3-Phenyl-isoxazol-5-yl, Oxazol-2-yl, 2-Methyloxazol-4-yl, Thiazol-4-yl, 2-Benzthiazol-4-yl, 4-Methylthiazol-2-yl, 4-Methylthioazol-5-yl, 4-Phenylthiazol-2-yl, 2-Phenylthiazol-5-yl.

Gegebenenfalls substituierte Thienyl- oder Benzthienylreste sind z.B. 2-Thienyl, 3-Thienyl, 2,3-Dichlor-4-thienyl, 2-Methyl-5-thienyl, 2-Nitro-5-thienyl, 2-Benzthienyl, 3-Benzthienyl.

Gegebenenfalls substituierte Naphthyl- oder Chinolinylreste sind z.B. 1-Naphthyl, 2-Naphthyl, 2-Chlor-1-naphthyl, 6-Chlor-1-naphthyl, 2,6-Dichlor-1-naphthyl, 2-Methyl-6-chlor-1-naphthyl, 2-Chinolinyl, 4-Chinolinyl, 6-Chinolinyl, 8-Chinolinyl, 6-Methyl-2-chinolinyl.

Wird ein Rest als "gegebenenfalls substituiert" bezeichnet, so kann er einen oder mehrere, insbesondere ein bis drei der folgenden Substituenten tragen: Halogen, insbesondere Chlor und Brom, Nitro, Cyano, sowie Alkyl, Halogenalkyl, Alkoxy und Alkylthio wie für R¹ genannt.

Insbesondere bevorzugt sind Verbindungen der Formel I, in denen der Rest R für Methoxy steht, X Sauerstoff bedeutet und Y sowie die Reste R¹, R³ und R⁴ die eingangs erwähnte Bedeutung haben.

Als Salze der Verbindungen der Formel I kommen umweltverträgliche Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden, wachstumsregulierenden, fungiziden oder nitrifikationshemmenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 3.008 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 3.013 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 3.008 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des AnlagerungsproduKtes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 3.008 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 3.008 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 3.013 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 3.013 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 3.008 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 1, vorzugsweise 0,01 bis 0,5 kg/ha aktive Substanz (a.S.).

Die wachstumsregulierend wirkenden Salicylsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) von dem Zeitpunkt der Applikation, bezogen auf das Entwicklungsstadium der Pflanze und von der Jahreszeit,
c) von dem Applikationsort und -verfahren z.B. (Samenbeize, Bodenbehandlung, Blattapplikation oder Stamminjektion bei Bäumen)
d) von klimatischen Faktoren, z.B. Temperatur, Niederschlagsmenge, außerdem auch Tageslänge und Lichtintensität
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel Ia im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Salicylsäurederivate der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g, je Kilogramm Saatgut, benötigt.

Für die Blatt- und Bodenbehandlung sind im allgemeinen Gaben von 0,001 bis 10 kg/ha, bevorzugt 0,01 bis 3 kg/ha, insbesondere 0,01 bis 0,5 kg/ha als ausreichend zu betrachten.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

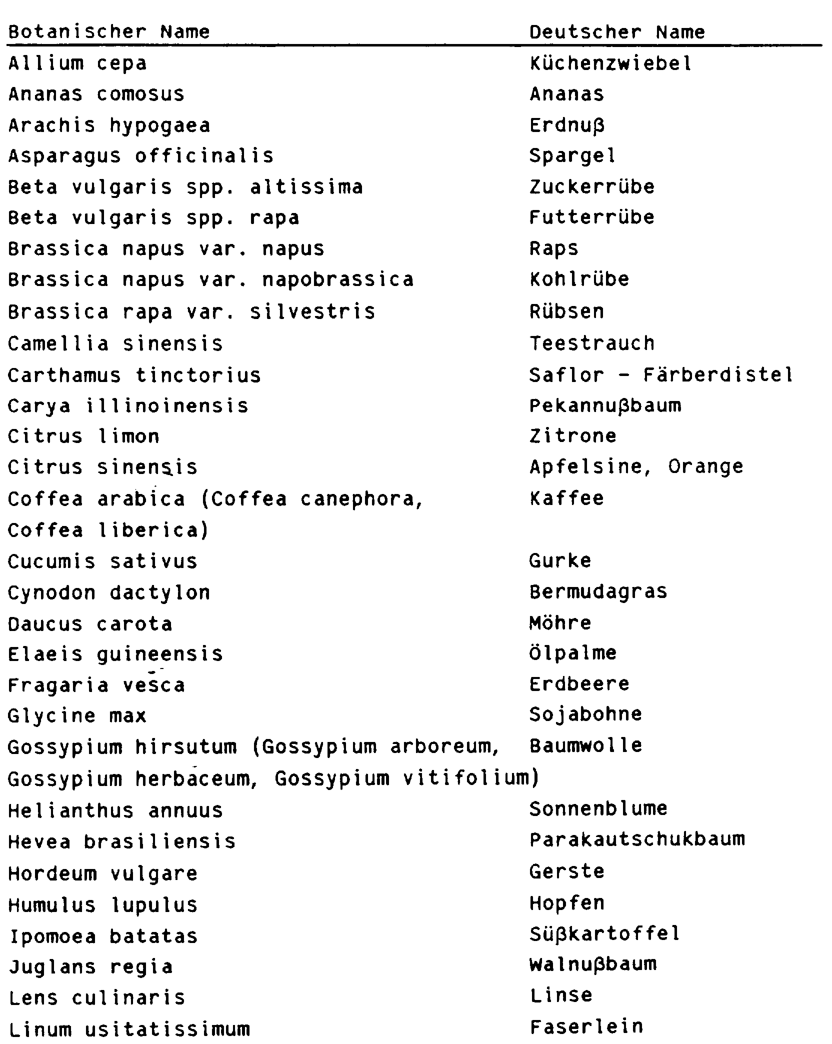

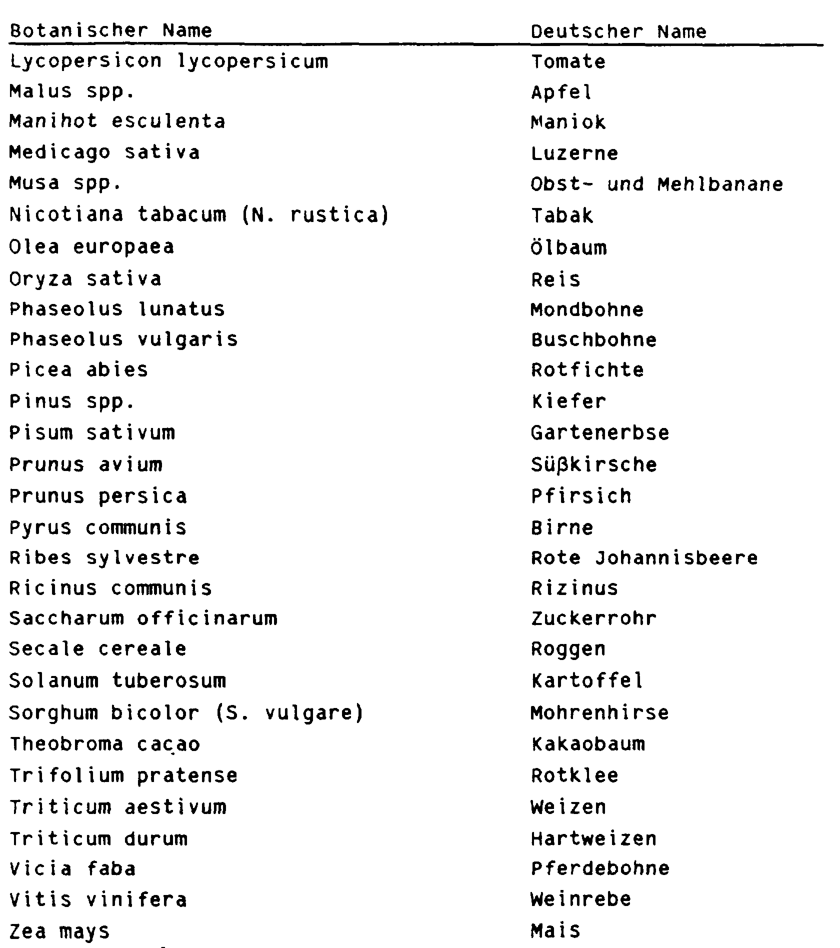

Weiterhin haben die Verbindungen I eine besondere Bedeutung für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wriksamen Menge der Wirkstoffe behandelt. Die Anwendung erfolgt vor oder nach der Infektion der Materialien, Pflanzen oder Samen durch die Pilze.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, säuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I bzw. II benutzt. Die so erhaltenen Verbindungen sind in den nachstehenden Tabellen mit physikalischen Angaben aufgeführt. Verbindungen ohne diese Angaben lassen sich aus den entsprechenden Edukten analog synthetisieren. Die in der Tabelle 3 wiedergegebenen Strukturen beschreiben besonders bevorzugte Wirkstoffe der Formel I.

### Beispiel 1

### 6-Phenylsalicylsäuremethylester:

### Variante 1

58 g (0,5 mol) Acetessigsäuremethylester gibt man zu einer Lösung von 0,5 g Natriumhydrid in 100 ml getrocknetem Ethanol, tropft 66 g (0,5 mol) Zimtaldehyd schnell zu und rührt ca. 10 h bei Raumtemperatur. Die Mischung wird dann bei 0°C mit trockenem HCl-Glas gesättigt und anschließend ca. 1 d bei Raumtemperatur (20°C) nachgerührt. Man entfernt das Lösungsmittel und destilliert den verbliebenen Rückstand im Vakuum, wobei zunächst HCI-Glas freigesetzt wird. Die von 115 bis 165°C/0,2 mbar übergehenden Fraktionen werden vereinigt und an Kieselgel (Eluation mit Toluol/Cyclohexan) chromatographiert. Man erhält 33,7 g eines gelben Öls als Zwischenprodukt. Man löst dieses in 150 ml Methylenchlorid und versetzt bei 0°C mit einer Lösung von 22,9 g (0,14 mol) Brom in 150 ml Eisessig zügig, wärmt langsam auf und kocht eine Stunde unter Rückfluß. Die Reaktionslösung wird zu 200 ml Methylenchlorid und 500 ml Wasser gegossen. Die organische Phase wird abgetrennt und in üblicher Weise aufgearbeitet. Man erhält einen gelben Feststoff als Zwischenprodukt, der zur Weiterverarbeitung in 400 ml Methyl-t.-butylether gelöst wird. Man versetzt mit 55 g (0,56 mol) Triethylamin und kocht 5 h unter Rückfluß. Anschliepend fügt man 300 ml Wasser zu, trennt die organische Phase ab und arbeitet in üblicher Weise auf. Der Rückstand wird bei 100 bis 114°C/0,2 mbar destilliert. Ausbeute: 11,9 g.

### Variante 2

Zur Lösung von 57 g (0,15 mol) 4-(Triphenylphosphoranyliden)-acetessigsäuremethylester in 700 ml THF gibt man portionsweise unter Stickstoffatmosphäre 8,55 g (0,3 mol) Natriumhydrid (85 %) und erwärmt danach auf ca. 35°C. Bei dieser Temperatur gibt man nun portionsweise 20 g (0,15 mol) Zimtaldehyd und anschließend 5 - 10 Tropfen Wasser hinzu, wobei die Reaktion anfangs exotherm verläuft und gekühlt werden muß. Es wird nun solange bei 35°C weitergeführt, bis das Ylid vollständig umgesetzt ist (DC-Kontrolle, ca. 12-14 h). Die Reaktionsmischung wird nun mit 10 %iger Salzsäure sauer gestellt und nach dem Zufügen von 1 l Wasser viermal mit je 150 ml Ether extrahiert. Die vereinten Etherphasen werden mit 200 ml wasser und 200 ml gesättigter Kochsalzlösung ausgeschüttelt und nach dem Trocknen über Natriumsulfat eingeengt. Der zurückbleibende schwarzbraune Rückstand wird mit 400 ml Methyl-tert.-butylether aufgenommen und mehrere Stunden gekocht. Nach dem Abkühlen filtriert man vom unlöslichen Rückstand (Triphenylphosphinoxid) ab und engt das Filtrat ein. Das zurückbleibende öl wird durch Chromatographie an Kieselgel gereinigt (Laufmittel: Toluol/Essigester mit nach und nach ansteigendem Essigester-Anteil). Alle Fraktionen vor dem Triphenylphosphinoxid werden vereint und analog obiger Vorschrift bromiert, aromatisiert und aufgearbeitet. Man erhält 14,5 g des Produktes.

Analog können beispielsweise auch die folgenden Verbindungen hergestellt werden:
6-(3,5-Dichlorphenyl)salicylsäuremethylester, ¹H-NMR (CDCl₃: δ = 3,58 (s; 3H); 6,70 (d; 1H); 7,05 (d; 1H); 7,10 (d; 2H); 7,25-7,40 (m; 2H); 10,85 (s; 1H).
6-(2,4-Dichlorphenyl)salicylsäuremthylester, ¹H-NMR (CDCl₃: δ = 3,58 (s; 3H); 6,65 (d; 1H); 7,0-7,5 (m; 5H); 11,1 (s; 1H).

### Beispiel 2

Allgemeine Vorschrift zur Herstellung der Salicylsäurederivate der Formel I:

0,073 mol der jeweiligen aromatischen 2-Hydroxycarbonsäure werden in 320 ml getrocknetem Dimethylsulfoxid gelöst und portionsweise mit 16,4 g (0,146 mol) Kalium-tert.-butylat versetzt, wobei die Temperatur der Reaktionsmischung auf etwa 30°C steigt. Man kühlt auf Raumtemperatur, gibt 16,8 g (0,073 mol) 4-Methoxy-2-methylsulfonyl-5,6-dihydrofuran[2,3-d]pyrimidin zu und rührt etwa 1 h bei Raumtemperatur nach. Die Reaktionsmischung wird in etwa 2 l kaltes Wasser gegossen, mit Salzsäure angesäuert und mit Methyl-tert.-butylether extrahiert. Nach üblicher Aufarbeitung kann das zurückbleibende Rohprodukt, wenn erforderlich, durch Verrühren mit einem geeigneten Lösungsmittel oder durch Chromatographie an Silica-Gel gereinigt werden.

### Beispiel 3

Allgemeine Vorschrift zur Herstellung der Salicylsäurederivate der Formel I:

5,1 g Kaliumhydroxid und 0,08 mol der jeweiligen 2-Hydroxycarbonsäure werden in 80 ml Methanol gelöst, 10 min bei Raumtemperatur gerüht und im Vakuum eingeengt. Anschließend wird zum Trocknen wiederholt mit Toluol versetzt und dieses bei 50°C im Vakuum verdampft. Das so erhaltene hellrote Pulver wird in 300 ml Dimethylsulfoxid aufgenommen und bei Raumtemperatur portionsweise mit 2,9 g 80 %igem Natriumhydrid versetzt, wobei eine Gasentwicklung auftritt. Wenn kein Gas mehr frei wird, tropft man eine Lösung von 0,08 Mol 4-Methoxy-2-methylsulfonyl-5,6-dihydrofuran[2,3-d]pyrimidin in 80 ml Dimethylsulfoxid zu und rührt 0,5 h nach. Man gießt in 2 l Wasser, neutralisiert mit Essigsäure und wäscht mit Methylenchlorid. Anschließend wird mit Salzsäure stark angesäuert und mehrmals mit Methyl-tert.-butylether extrahiert. Die organische Phase trocknet man über Natriumsulfat und verdampft das Lösungsmittel im Vakuum. Die zurückbleibende Substanz kann durch Chromatograhie an Silica-Gel gereinigt werden.

### Beispiel 4

Allgemeine Vorschrift zur Herstellung aromatischer Carbonsäureoximester oder ähnlicher Verbindungen der Formel I:

3,2 mmol der jeweiligen aromatischen 2-(4-Methoxy-5,6-dihydrofuran[2,3-d]pyrimidin-2-yl)oxycarbonsäure werden in 20 ml Diemthoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1 h nach und verdampft dann zur Entfernung des überschüssigen Oxalylchlorids etwa 30 % des Lösungsmittel im Vakuum. Man gibt nun 4,2 mmol des jeweiligen Oxims oder einer vergleichbaren Hydroxy-Verbindung gelöst in 10 ml Dimethoxyethan und anschließend 3,2 mmol Pyridin bei 0°C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Die zurückbleibende Substanz kann durch Chromatographie an Silica-Gel weiter gereinigt werden.

### Beispiel 5

a) 2-Methoxy-6-(pyrazol-1-yl)-benzonitril:
   Zu einer Lösung von 41,2 g (0,273 mol) 2-Methoxy-6-fluorbenzonitril (Herstellung: J. Heterocycl. Chem. 25, 1173 (1988) in 50 ml N,N-Dimethylethylenharnstoff werden unter Stickstoff bei 50°C eine Lösung von 0,273 mol Natriumpyrazolid (hergestellt aus äquimolaren Mengen Pyrazol und Natriumhydrid) in 140 ml N,N-Dimethylethylenharnstoff zugetropft und 2 h bei 60°C nachgerührt. Nach dem Abkühlen und Einrühren des Ansatzes in 4 l Eiswasser, Absaugen und Trocknen der ausgefallenen Kristalle erhält man 37,1 g des Produktes vom Schmelzpunkt 93-94°C.
b) 2-Methoxy-6-(pyrazol-1-yl)benzoesäure-O-ethyliminoester:
   In 68,0 g einer 30 %igen Lösung von trockenem HCl-Glas in Ethanol werden unter Rühren 37,1 g (0,186 mol) 2-Methoxy-6-(pyrazol-1-yl)benzonitril bei 0°C unter Ausschluß von Feuchtigkeit eingetragen. Nach Verdünnen mit 20 ml Ethanol wird das Gemisch 48 h bei Raumtemperatur gerührt, in 500 ml Eiswasser gegeben und durch Zugabe von 2-normaler Natronlauge und dann gesättigter Natriumhydrogencarbonat-Lösung auf einen pH-Wert von 7 eingestellt. Nach dem Absaugen und Trocknen der ausgefallenen Kristalle erhält man 30,3 g des Produktes vom Schmelzpunkt 72-73°C.
c) 2-Methoxy-6-(pyrazol-1-yl)benzoesäureethylester:
   29,0 g (0,118 mol) 2-Methoxy-6-(pyrazol-1-yl)benzoesäure-O-ethyliminoester und 300 ml Salzsäure werden 16 h bei 50°C gerührt. Nach dem Abkühlen wird das Reaktionsgemisch 3mal mit je 100 ml Methylenchlorid extrahiert. Nach dem Einengen des Extraktes erhält man 16,7 g Rückstand, der an Kieselgel mit Toluol/Essigsäureethylester (9:1) chromatographiert wird. Man erhält 13,4 g des Produktes vom Schmelzpunkt 159-163°C.
d) 6-(Pyrazol-1-yl)-salicylsäureethylester:
   Zu einer Lösung von 15,3 g (0,062 mol) 2-Methoxy-6-(pyrazol-1-yl)benzoesäureethylester in 140 ml Methylenchlorid werden bei 20 bis 25°C 200 ml (0,2 mol) 1-molare Bortribromid-Lösung zugetrpft. Man rührt ca. 10 h bei Raumtemperatur und tropft dann bei 0°C 160 ml Ethanol zu. Man rührt 15 min nach, entfernt dann das Lösungsmittel bei 30°C weitergehend im Vakuum und verrührt den Rückstand mit 200 ml Wasser. Man extrahiert den Rückstand 3mal mit je 70 ml Diethylether, engt ein und chromatographiert das Rohprodukt an Kieselgel mit Toluol/Essigsäureethylester. Ausbeute: 8,7 g des Produktes als Öl. ¹H-NMR (ausgewählte Signale): δ = 0,98 (t); 4,10 (q); 6,38; 6,90; 7,10; 7,45 (t); 7,60; 7,70; 10,70 (s).
   Aus dieser Verbindung läßt sich 6-(Pyrazol-1-yl)-salicylsäure durch Hydrolyse mit verdünnter Natronlauge gewinnen (Schmelzpunkt: 175-179°C).

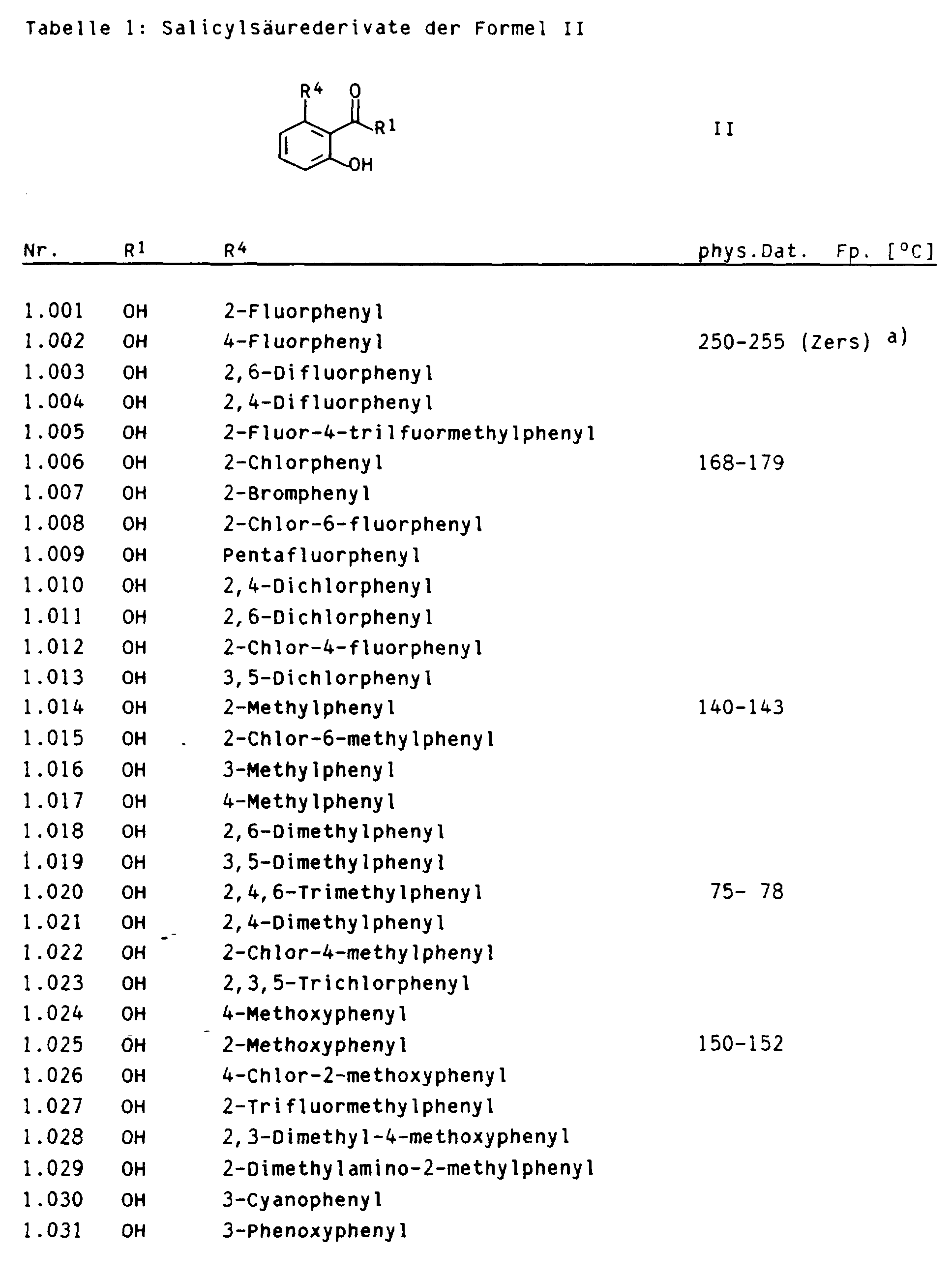

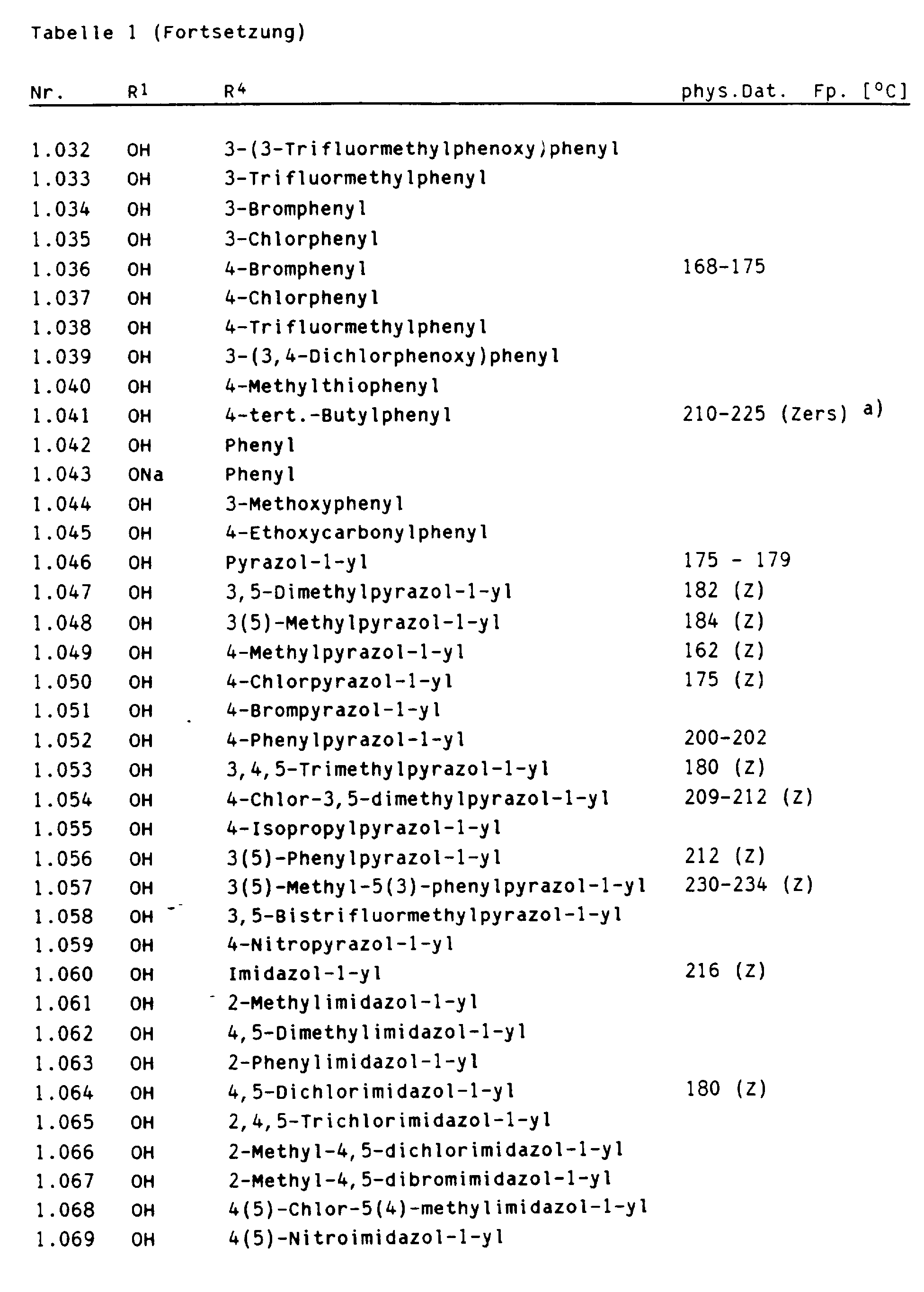

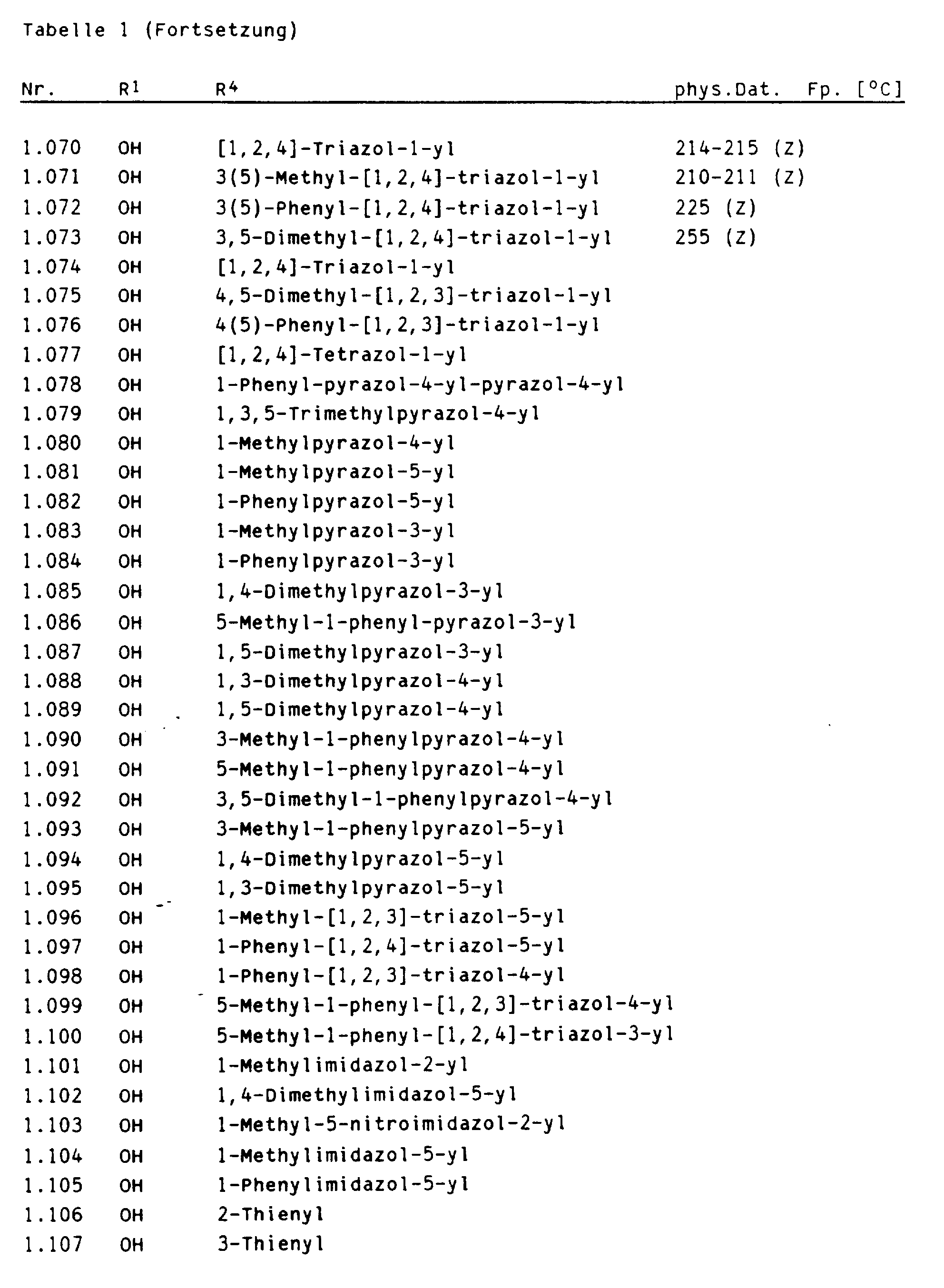

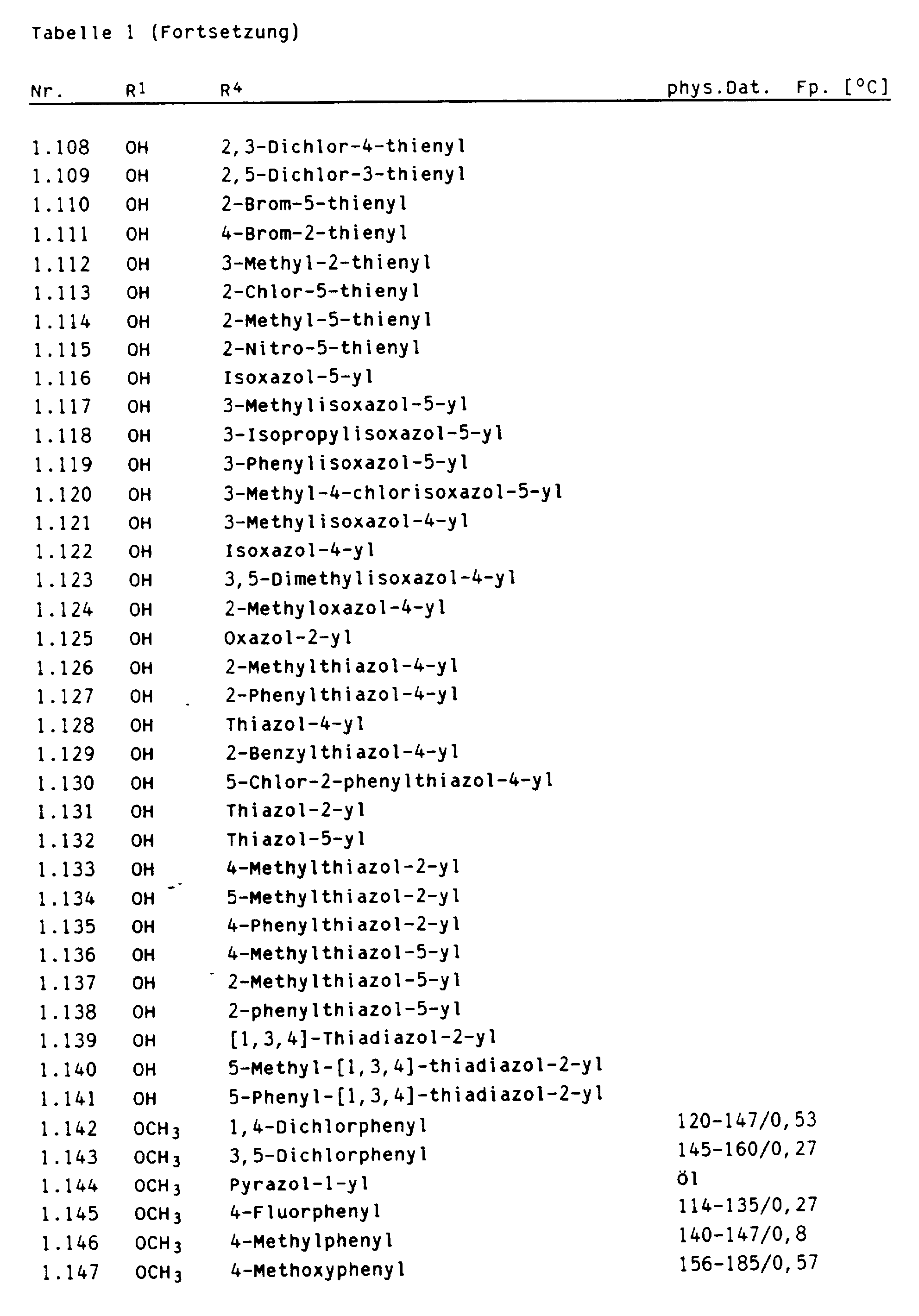

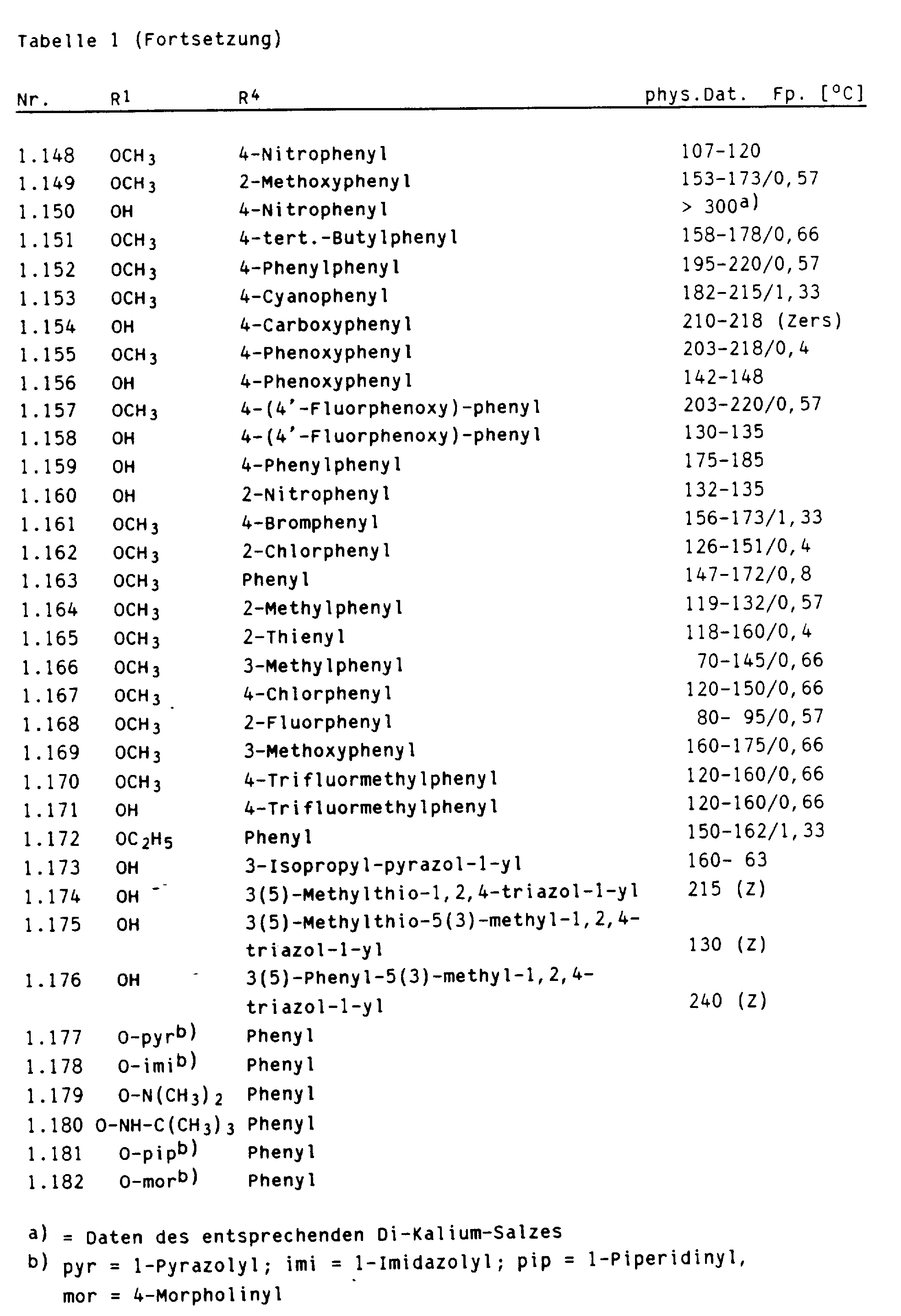

### Beispiel 6

Herstellung von 2-Methylsulfonyl-4-methoxy-5,6-dihydrofuran[2,3-d]-pyrimidin

2-Methylthio-4-chlor-5,6-dihydrofuran[2,3-d]pyrimidin

Zu einer Suspension von 65,8 g (0,357 mol) 2-Methylthio-4-hydroxy-5,6-dihydrofuran[2,3-d]pyrimidin (Collect. Czech. Chem. Commun. 32, 1582 (1967)) in 900 ml Chlorbenzol tropft man bei 125-130°C 212,0 g (1,07 mol) Trichlormethylchlorformiat in 3 Std. zu, wobei dreimal je 0,5 ml DMF zugesetzt wird. Nach Istündigem Rühren bei 130°C wird das Reaktionsgemisch im Vakuum eingeengt und der Rückstand (74 g öl) an Kieselgel chromatographiert (Toluol-Cyclohexan-Gemisch 9:1). Ausbeute: 17,0 g des o.g. Produkts vom Fp. 68-71°C.

2-Methylthio-4-methoxy-5,6-dihydrofuran[2,3-d]pyrimidin

17,0 g (84 mmol) 2-Methylthio-4-chlor-5,6-dihydrofuran[2,3-d]pyrimidin werden in 90 ml Methanol gegeben, bei 45°C 21,1 g (0,117 mol) 30 %ige Natriummethylat-Lösung zugetropft und 2 Std. bei 50°C nachgerührt. Nach Neutralisieren auf pH 6 mit etwas Eisessig wird das Reaktionsgemisch in 350 ml Eiswasser eingerührt. Nach Absaugen, Waschen mit Wasser und Trocknen erhält man 15,1 g des o.g. Produkts vom Fp. 90-92°C.

2-Methylsulfonyl-4-methoxy-5,6-dihydrofuran[2,3-d]pyrimidin

In eine Mischung von 15,1 g (76 mmol) 2-Methylthio-4-methoxy-5,6-dihydrofuran[2,3-d]pyrimidin in 120 ml Methylenchlorid und 76 ml Wasser leitet man bei 0 bis 5°C unter Rühren Chlor ein, bis die Reaktionsmischung blaß gelb gefärbt ist. Nach 30minütigem Nachrühren wird die organsche Phase abgetrennt und die Wasserphase mit 100 ml Methylenchlorid extrahiert. Die vereinigten organischen Phasen werden getrocknet und eingeengt. Aus dem Rückstand (16,7g) isoliert man nach Chromatographie an Kiesel (Toluol-Essigester-Gemisch 4:1) 5,5 g des o.g. Produkts vom Fp. 122-24°C.

### Beispiel 7

Herstellung von 2-Methylsulfonyl-4-methyl-5,6-dihydrofuran[2,3-d]pyrimidin

Analog zu Beispiel 6 erhält man aus 2-Methylthio-4-methyl-5,6-dihydrofuran[2,3-d]pyrimidin (Collect. Czech. Chem. Commun. 32, 1582 (1967)) das obige Produkt vom Fp. 85-90°C in 80 % Ausbeute.

In entsprechender Weise sind die in Tabelle 2 aufgeführten Sulfone III erhältlich.

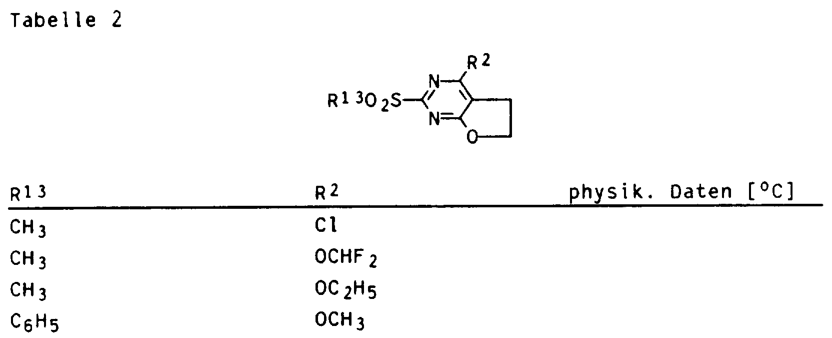

### Beispiel 8

3-(4-Methoxy-5,6-dihydrofuran[2,3-d]pyrimidin-2-yl)oxy-biphenyl-2-carbonsäure (Verbindung Nr. 5.001):

Zu 1,50 g (7 mmol) 6-Phenylsalicylsäure in 15 ml trockenem Dimethylsulfoxid werden 1,57 g (14 mmol) Kalium-tert.-butylat zugegeben und 1 Stunde bei Raumtemperatur gerührt. Nach Zugabe von 1,61 g (7 mmol) 2-Methylsulfonyl-4,6-dimethoxy-5,6-dihydrofuran[2,3-d]-pyrimidin wird das Reaktionsgemisch 48 Stunden bei Raumtemperatur gerührt und dann auf 300 ml Wasser, dem 2,5 ml Phosphorsäure zugesetzt sind, gegeben. Der Niederschlag wird isoliert, mit Toluol angeteigt und abgesaugt. Aus dem Rohprodukt erhält man durch einstündiges Rühren in einer Mischung von 0,5 ml Phosphorsäure und 10 ml Wasser nach Absaugen und Trocknen 1,3 g des obigen Produkts vom Fp.: 176-178°C.

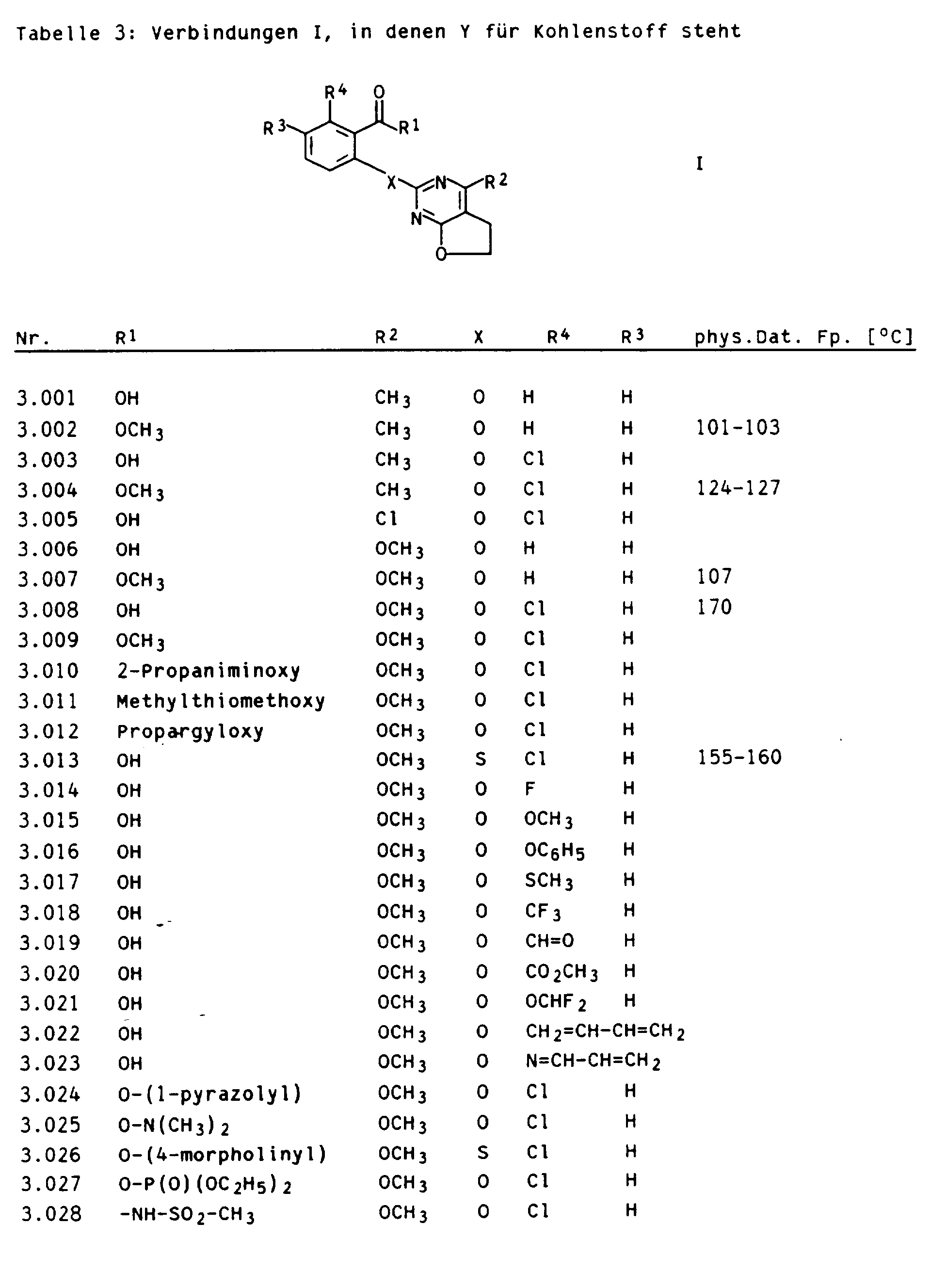

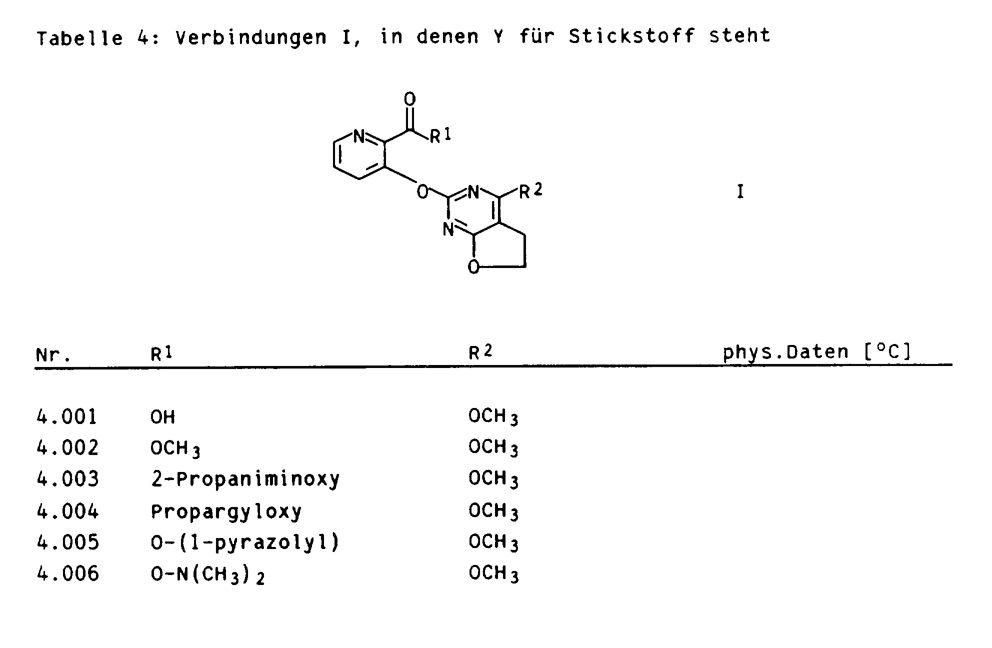

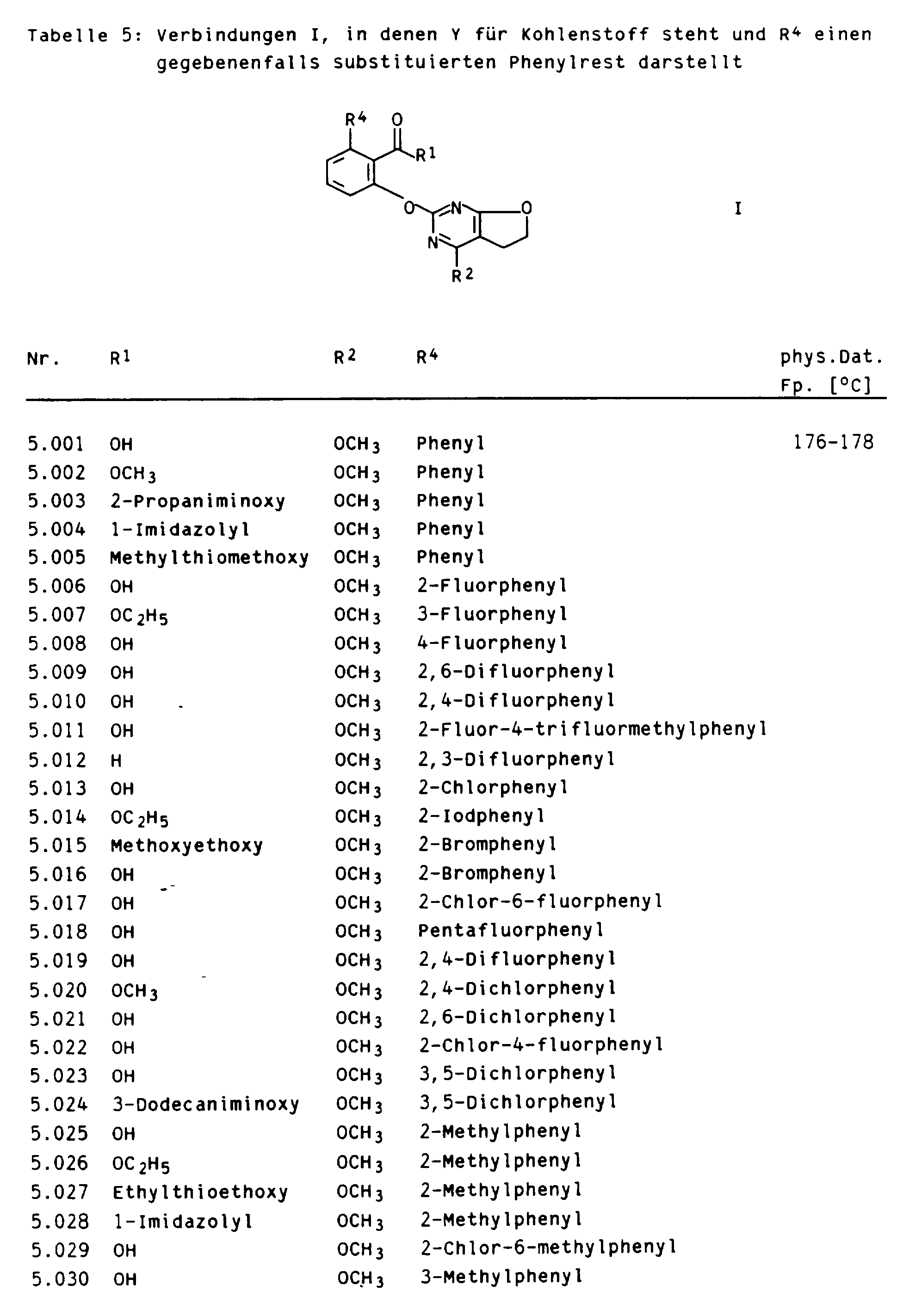

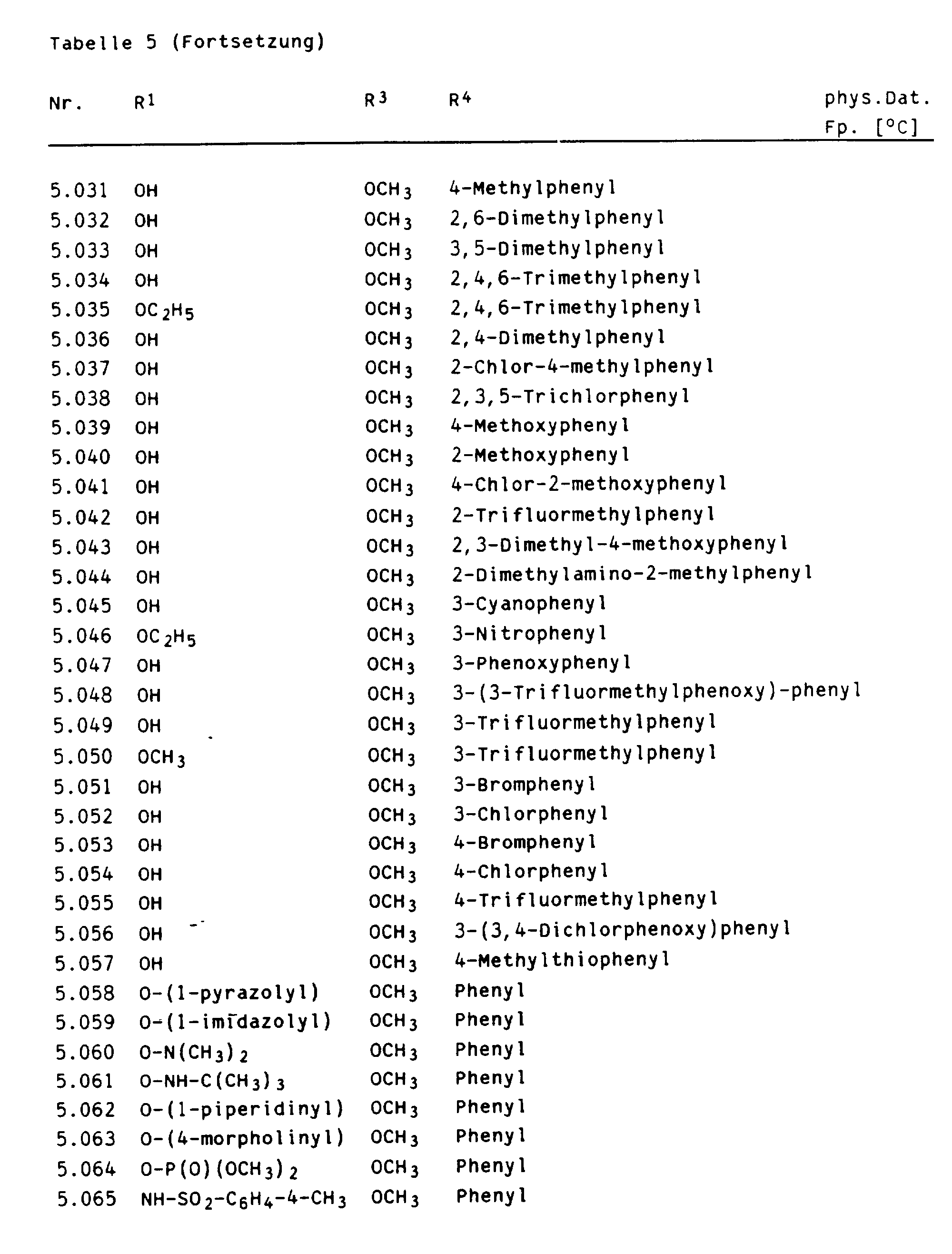

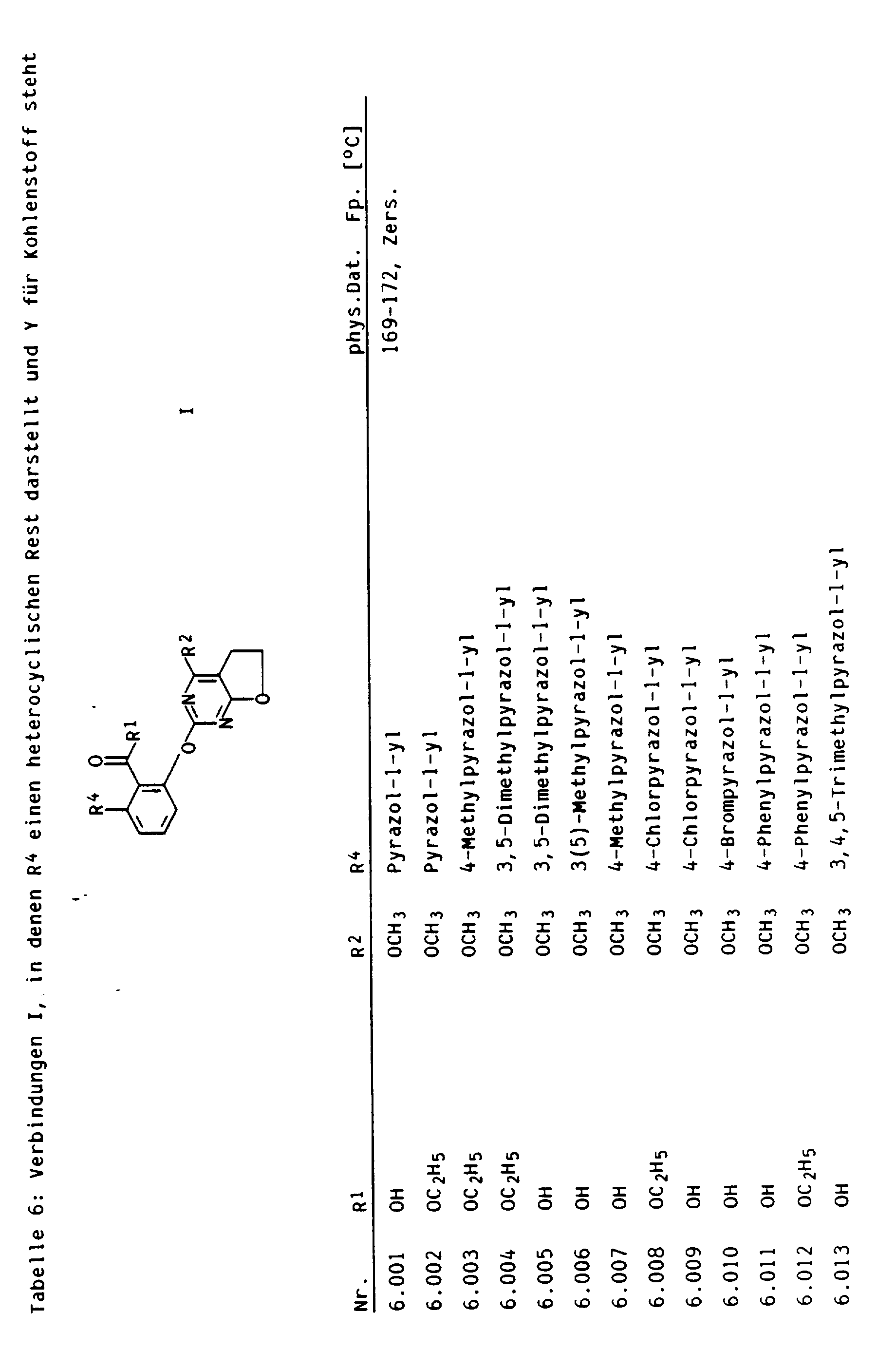

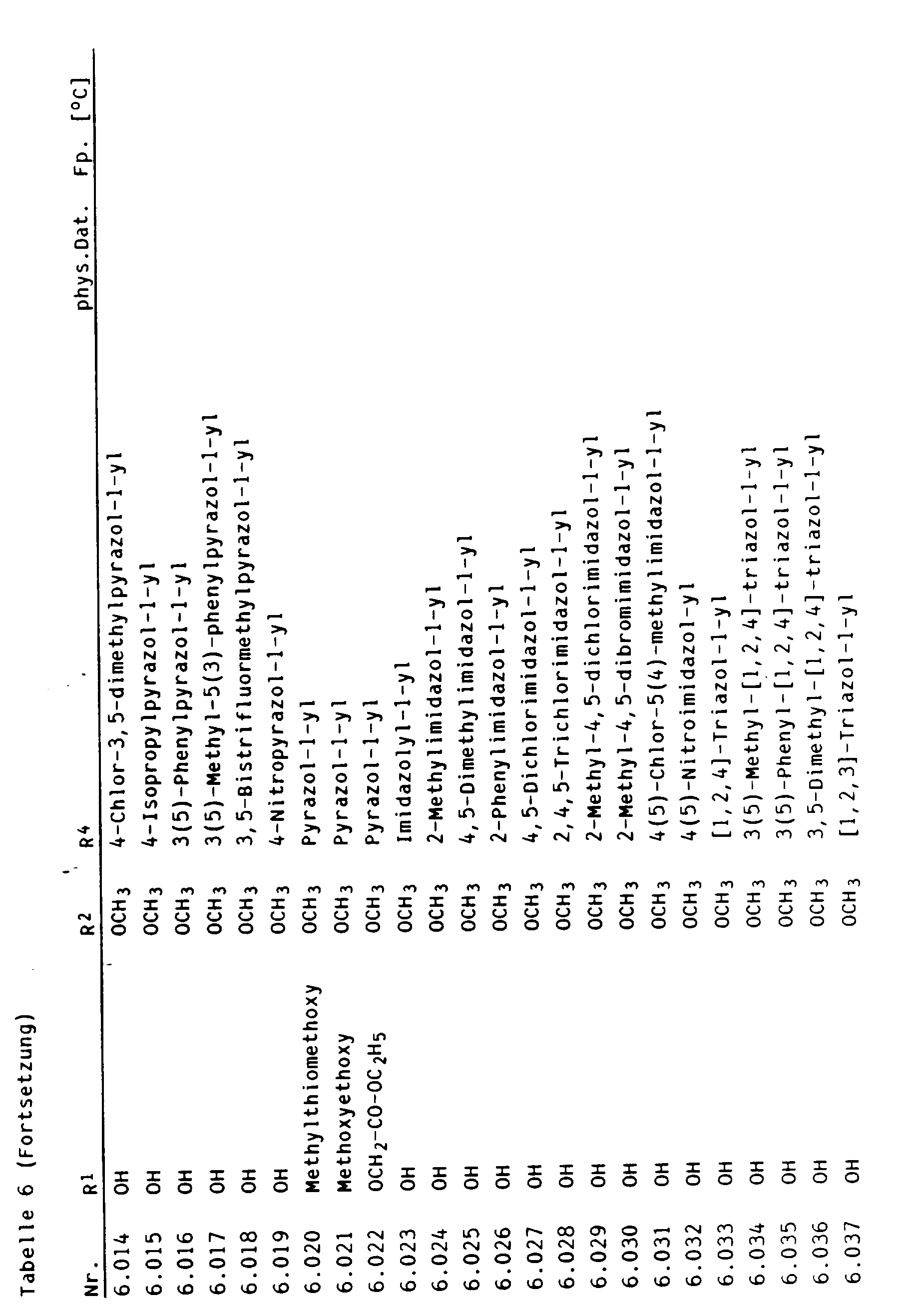

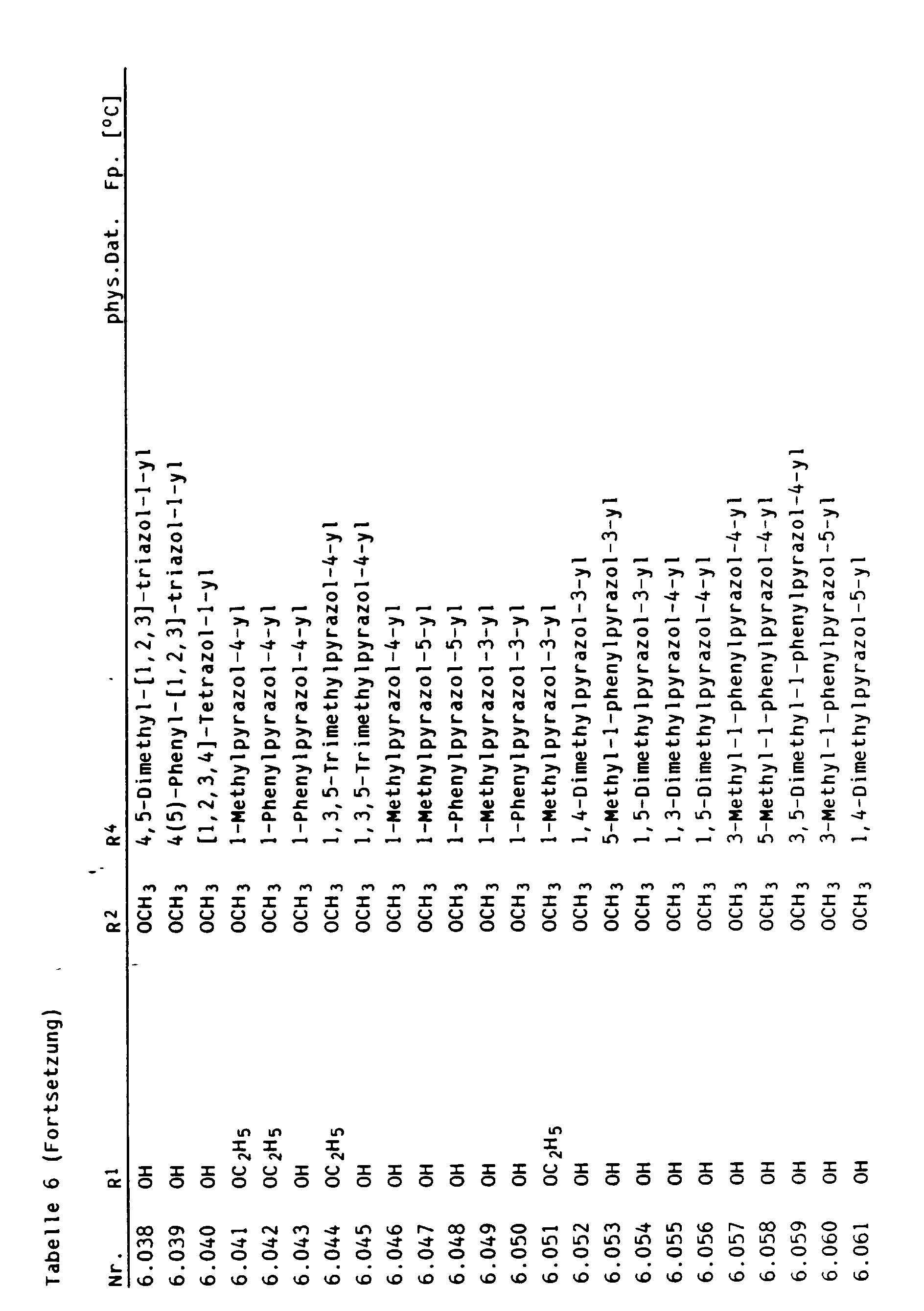

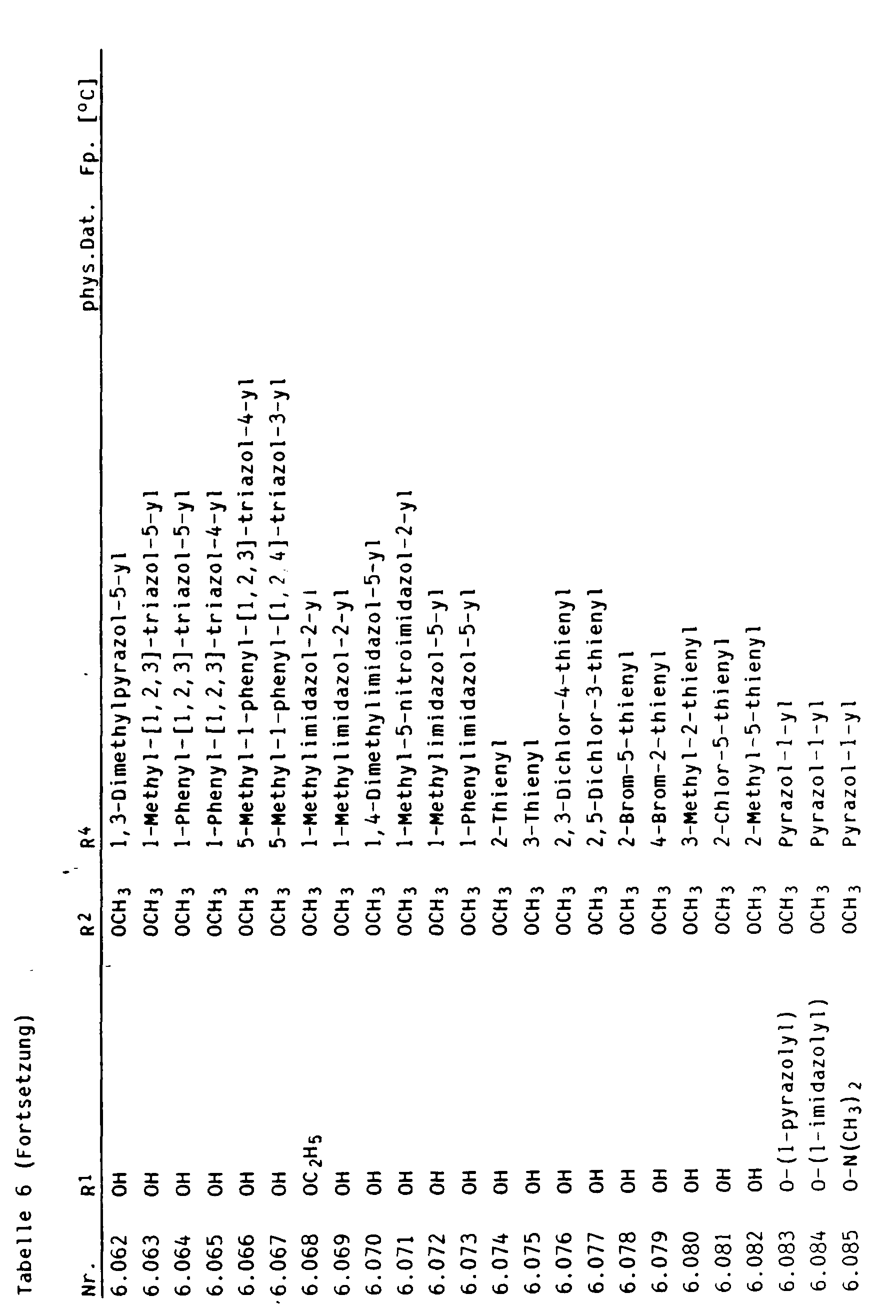

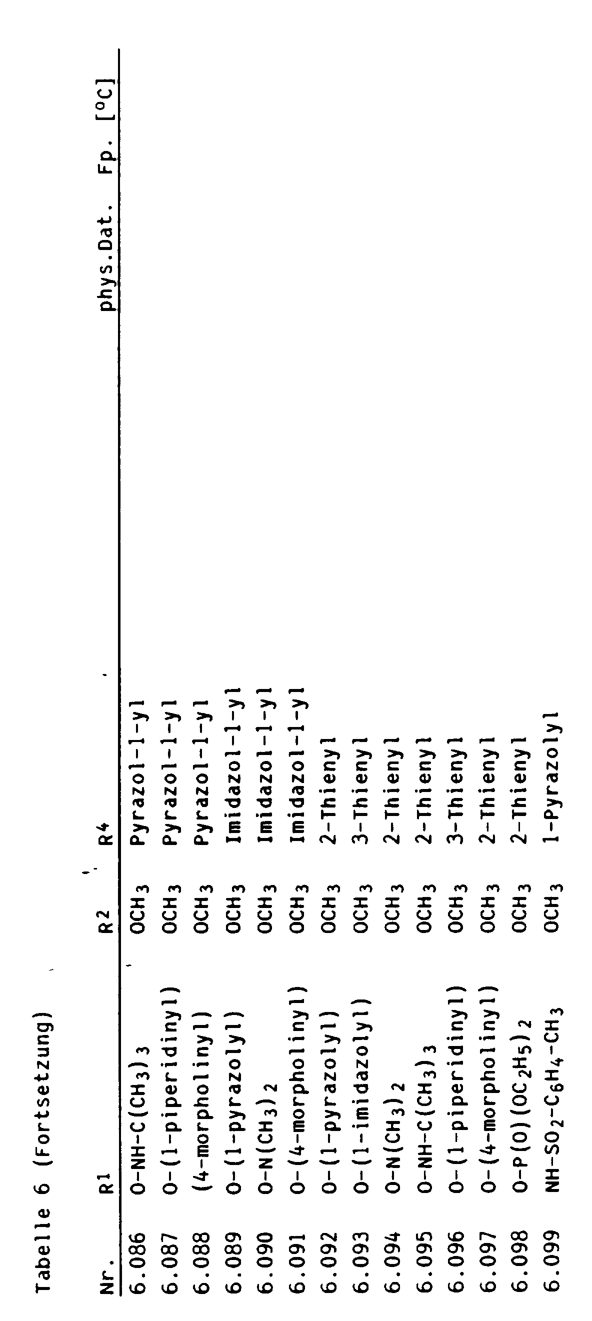

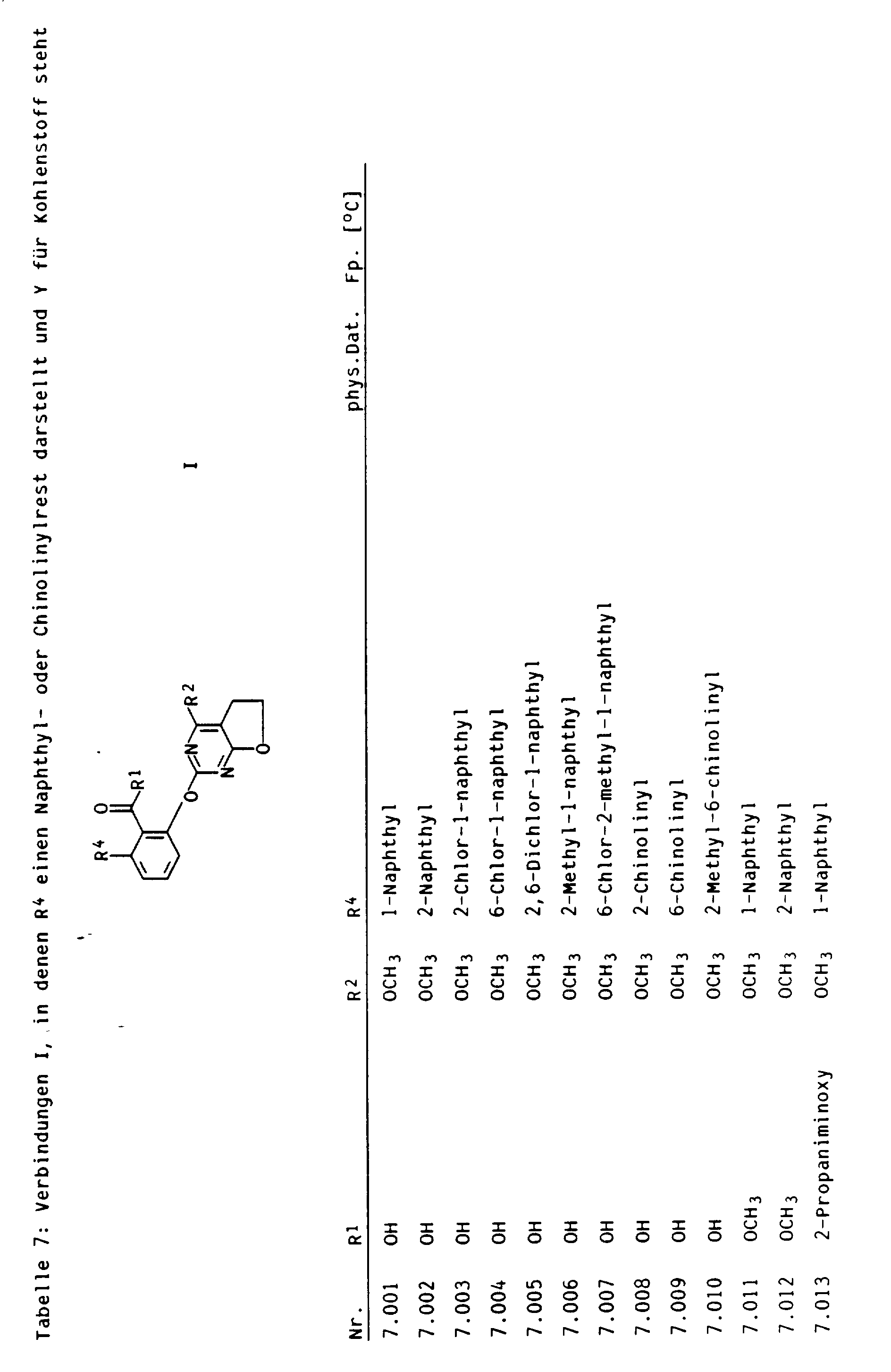

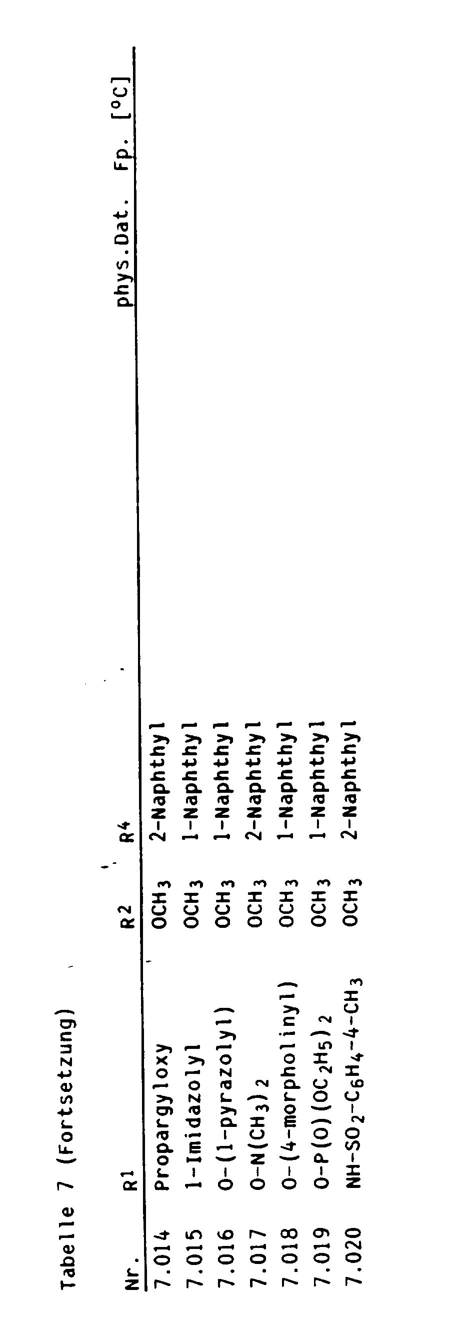

Anwendungsbeispiele zur herbiziden Wirkung

Die herbizide Wirkung der Carbonsäurederivate der Formel I ließ sich durch Gewächshausversuche zeigen:

Zur Aufzucht der Testpflanzen dienten Plastikblumentöpfe mit 300 cm³ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung betrug 0,06 kg Wirkstoff/ha a.S. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| ABUTH | Abutilon theophrasti | Chinesischer Hanf |
| AMARE | Amaranthus retroflexus | Zurückgekrümmter Fuchsschwanz |
| SOLNI | Solanum nigrum | Schwarzer Nachtschatten |

Mit 0,06 kg/ha a.S. im Nachauflaufverfahren eingesetzt, lassen sich mit den Beispielen 3.008 und 3.013 unerwünschte Pflanzen sehr gut bekämpfen.

## Patentansprüche

1. Salicylaldehyd- und Salicylsäurederivate und deren Schwefelanaloge der Formel I, 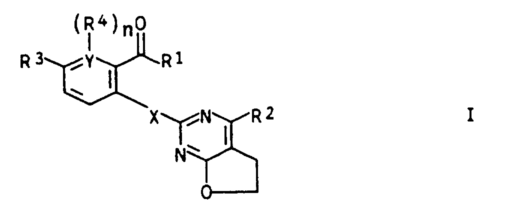 in der die Substituenten folgende Bedeutung haben:
R¹ Wasserstoff
eine Succinylimidooxygruppe;
ein 5-gliedriger Heteroaromat, enthaltend ein bis drei Stickstoffatome, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
einen Rest -OR⁵, worin
R⁵
Wasserstoff, ein Alkalimetallkation, das Äquivalent eines Erdalkalimetallkations oder ein organisches Ammoniumion;
eine C₃-C₁₂-Cycloalkylgruppe, welche ein bis drei C₁-C₄-Alkylreste tragen kann;
eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, C₃-C₁₂-Cycloalkyl, Phenyl, Phenoxy oder Phenylcarbonyl, wobei die aromatischen Reste ihrerseits ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein 5-gliedriger Heteroaromat, enthaltend ein bis drei Stickstoffatome oder ein Stickstoffatom und ein Sauerstoff- oder Schwefelatom, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
eine C₂-C₆-Alkylgruppe, welche in der 2-Position einen der folgenden Reste trägt: C₁-C₆-Alkoxyimino, C₃-C₆-Alkenyloxyimino, C₃-C₆-Halogenalkenyloxyimino oder Benzyloxyimino;
eine C₃-C₆-Alkenyl- oder eine C₃-C₆-Alkinylgruppe, wobei diese Gruppen ihrerseits ein bis fünf Halogenatome tragen können;
unsubstituiertes oder ein- bis dreifach durch C₁-C₄-Alkyl oder C₁-C₄-Alkoxy substituiertes oder ein- bis fünffach durch Halogen substituiertes Phenyl;
ein über ein Stickstoffatom gebundener 5-gliedriger aromatischer Heterocyclus mit ein bis vier Stickstoffatomen im Ring, welcher ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
eine Gruppe -N=CR⁶R⁷, worin
R⁶ und R⁷ C₁-C₂₀-Alkyl, welches einen Phenylrest, eine C₁-C₄-Alkoxy- und/oder eine C₁-C₄-Alkylthiogruppe tragen kann, Phenyl oder gemeinsam eine C₃-C₁₂-Alkylenkette, welche ein bis drei C₁-C₃-Alkylgruppen tragen kann, bedeuten;
einen Rest 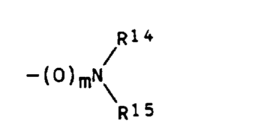 in dem m die Werte 0 und 1 annimmt und R¹⁴ und R¹⁵, die gleich oder unterschiedlich sein können, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthia, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, bis-C₁-C₆-Dialkylamino, C₃-C₆-Cycloalkyl, Phenyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
C₃-C₆-Cycloalkyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
Phenyl, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio;
R⁶ zusammen mit R⁷ eine 4- bis 7-gliedrige Alkylenkette, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio und in der eine CH₂-Gruppe durch Sauerstoff, Schwefel oder -NH ersetzt sein kann;
eine Gruppe 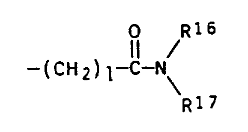 in der R¹⁶ und R¹⁷ für Wasserstoff, C₁-C₆-Alkyl, C₃-C₆-Alkenyl oder C₃-C₆-Alkinyl stehen und 1 die Werte 1, 2, 3 oder 4 bedeutet;
oder eine Gruppe
-(CH₂)ₗ-S(=O)ₖ-R¹⁸
in der R¹⁸ für C₁-C₆-Alkyl, C₃-C₆-Alkenyl oder C₃-C₆-Alkinyl, l für 1, 2, 3 oder 4 und k für 0, 1 oder 2 stehen;
einen Rest OR⁵, worin R⁵ eine Grüppe 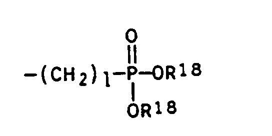 darstellt, in der R¹⁸ und l die obengenannte Bedeutung haben;
oder einen Rest 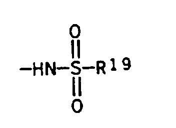 in dem R¹⁹ für die Reste C₁-C₆-Alkyl oder Phenyl steht, die ihrerseits ein bis vier der folgenden Substituenten tragen können; Halogen, Nitro, Cyano, C₁-C₆-Alkyl;
R C₁-C₄-Alkyl, C₁-C₄-Alkoxy, C₁-C₄-Halogenalkoxy, oder Halogen;
R³ Wasserstoff oder zusammen mit R⁴ eine Gruppe CH₂=CH-CH=CH₂ oder N=CH-CH=CH₂;
R⁴ Wasserstoff, Halogen, C₁-C₄-Alkoxy, C₁-C₄-Halogenalkoxy, Aryloxy, C₁-C₄-Halogenalkyl, Formyl, C₁-C₈-Alkoxycarbonyl;
einen gegebenenfalls ein bis dreifach durch Halogen, Nitro, Cyan, C₁-C₆-Alkyl, C₁-C₆-Halogenalkyl, C₁-C₆-Alkoxy, C₁-C₆-Alkylthio substituierten, im Fall von Halogen als Substituent ein- bis fünffach durch Halogen, Nitro, Cyan, C₁-C₆-Alkyl, C₁-C₆-Halogenalkyl, C₁-C₆-Alkoxy, C₁-C₆-Alkylthio substituierten Phenylrest 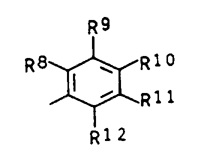
worin R⁸ - R¹
Wasserstoff, Halogen, Cyano, Nitro;
eine C₃-C₆-Alkenyl-, C₃-C₆-Alkenyloxy-, C₃-C₆-Alkinyloxy- oder eine C₃-C₆-Alkinylgruppe, wobei diese Gruppen ihrerseits ein bis fünf Halogenatome tragen können;
Di-C₁-C₄-alkylamino, C₃-C₈-Cycloalkyl, welches ein bis drei C₁-C₄-Alkylreste tragen kann;
C₁-C₁₀-Alkoxycarbonyl, C₁-C₄-Alkylthio;
eine Phenoxygruppe, wobei der aromatische Rest ein bis fünf Halogenatome und/oder ein bis drei der folgenden Reste tragen kann, C₁-C₄-Alkyl, C₁-C₄-Halogenalkyl, C₁-C₄-Halogenalkoxy, C₁-C₄-Alkoxy, C₁-C₄-Alkylthio;
eine C₁-C₁₀-Alkyl- oder Alkoxygruppe, welche ein bis fünf Halogenatome und/oder einen der folgenden Reste tragen kann: C₁-C₄-Alkoxy, C₁-C₄-Alkylthio, Phenyl oder Phenoxy, wobei die aromatischen Reste ihrerseits ein bis fünf Halogenatome und/oder ein bis drei der folgenden Reste tragen können: C₁-C₄-Alkyl, C₁-C₄-Halogenalkyl, C₁-C₄-Halogenalkoxy, C₁-C₄-Alkylthio;
bedeuten;
einen 5-gliedrigen Heteroaromaten, mit ein bis vier Stickstoffatomen oder ein bis zwei Stickstoffatomen sowie zusätzlich einem Schwefel- oder Sauerstoffatom im Ring oder einen benzokondensierten 5-gliedrigen Heteroaromaten mit ein bis drei Stickstoffatomen oder einem Stickstoffatom und zusätzlich einem Sauerstoff- oder Schwefelatom im Ring, welche ein bis drei Halogenatome und/oder einen bis drei der folgenden Reste tragen können: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
einen Thienyl- oder Benzthienylrest, der ein bis drei Halogenatome und/oder einen bis drei der folgenden Reste tragen kann: C₁-C₄-Alkyl, C₁-C₂-Halogenalkyl oder Nitro;
einen Naphthyl- oder Chinolinylrest, das ein bis drei der folgenden Substituenten tragen kann: Halogen, Nitro, Cyano, C₁-C₆-Alkyl, C₁-C₆-Halogenalkyl, C₁-C₆-Alkoxy, C₁-C₆-Alkylthio; oder Sauerstoff, wenn Y für Stickstoff und n für 1 steht;
n 1, wenn Y Kohlenstoff bedeutet oder wenn Y Stickstoff und R⁴ Sauerstoff bedeuten und 0, wenn Y für Stickstoff steht und R⁴ nicht Sauerstoff bedeutet;
X Sauerstoff oder Schwefel;
Y Stickstoff, wenn R³ Wasserstoff und n 0 bedeuten oder wenn R⁴ für Sauerstoff steht und n 1 bedeutet; Kohlenstoff, wenn R³ und R⁴ gemeinsam für eine Gruppe CH₂=CH-CH=CH₂ oder N=CH-CH=CH₂ stehen oder Kohlenstoff, wenn R³ Wasserstoff und n 1 bedeuten;
sowie umweltverträgliche Salze der Verbindungen I.

2. Salicylaldehyd- und Salicylsäurederivate der Formel I gemäß Anspruch 1, in der R Methoxy und X Sauerstoff bedeuten und Y sowie die Reste R¹, R³ und R⁴ die in Anspruch 1 genannte Bedeutung haben.

3. Herbizides Mittel, enthaltend eine Verbindung der Formel I gemäß Anspruch 1 und übliche inerte Zusatzstoffe.

4. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

5. Mittel zur Beeinflussung des Pflanzenwachstums, enthaltend ein Salicylaldehyd- oder Salicylsäurederivat der Formel I gemäß Anspruch 1 und übliche inerte Zusatzstoffe.

6. Verfahren zur Regulierung des Pflanzenwachstums, dadurch gekennzeichnet, daß man eine bioregulatorisch wirksame Menge eines Salicylaldehyd- bzw. Salicylsäurederivats der allgemeinen Formel I gemäß Anspruch 1 auf die Samen, die Pflanzen und/oder deren Lebensraum einwirken läßt.

7. Fungizides Mittel, enthaltend eine Verbindung der Formel I gemäß Anspruch 1 und übliche inerte Zusatzstoffe.

8. Neue Sulfone der allgemeinen Formel III 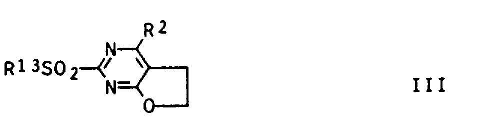 in der die Substituenten folgende Bedeutung haben:
R C₁-C₄-Alkyl, C₁-C₄-Alkoxy, C₁-C₄-Halogenalkoxy oder Halogen
R¹³ C₁-C₁₂-Alkyl, unsubstituiertes oder durch Alkyl, Halogen oder Alkoxy substituiertes Phenyl.

9. Verfahren zur Herstellung der Verbindungen der Formel I gemäß Anspruch 1, dadurch gekennzeichnet, daß man ein entsprechendes Salicylsäure- oder Thiosalicylsäurederivat der Formel II 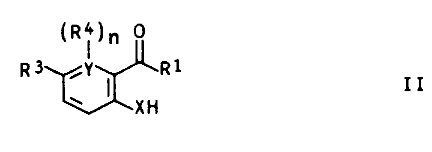 in an sich bekannter Weise mit einem Sulfon der Formel III, 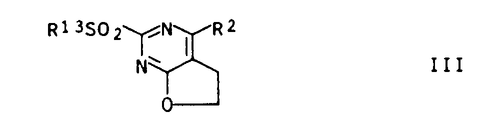 worin R¹³SO₂ für eine nucleofuge Abgangsgruppe steht, in Gegenwart einer anorganischen oder organischen Base umsetzt.

10. Verfahren zur Herstellung von Sulfonen der Formel III gemäß Anspruch 8, dadurch gekennzeichnet, daß man ein entsprechendes 2-Alkylthio- oder 2-Phenylthio-5,6-dihydrofuran[2,3-d]pyrimidin 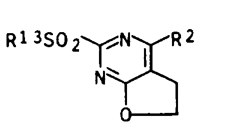 unter üblichen milden Bedingungen mit einem Oxidationsmittel behandelt.

## Claims

1. A salicylaldehyde or salicylic acid derivative or a sulfur analog thereof of the formula I 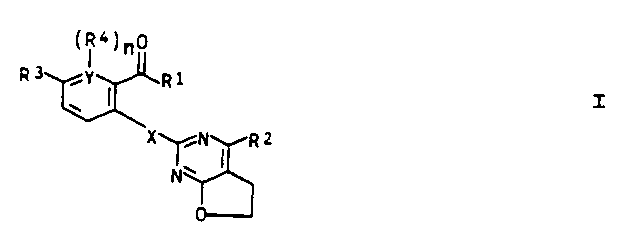 where
R¹ is hydrogen;
a succinylimidooxy group;
a 5-membered heteroaromatic structure which contains from one to three nitrogen atoms and may carry from one to four halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
a radical -OR⁵, where
R⁵ is hydrogen, an alkali metal cation, one equivalent of an alkaline earth metal cation or an organic ammonium ion;
C₃-C₁₂-cycloalkyl which may carry from one to three C₁-C₄-alkyl radicals;
C₁-C₁₀-alkyl which may carry from one to five halogen atoms and/or one of the following radicals: C₁-C₄-alkoxy, C₁-C₄-alkylthio, cyano, C₁-C₈-alkylcarbonyl, C₁-C₈-alkoxycarbonyl, C₃-C₁₂-cycloalkyl, phenyl, phenoxy or phenylcarbonyl, where the aromatic radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₁-C₁₀-alkyl which may carry from one to five halogen atoms and carries one of the following radicals: a 5-membered heteroaromatic structure which contains from one to three nitrogen atoms or one nitrogen atom and one oxygen or sulfur atom and may carry from one to four halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
C₂-C₆-alkyl which carries one of the following radicals in the 2-position: C₁-C₆-alkoximino, C₃-C₆-alkenyloximino, C₃-C₆-haloalkenyloximino or benzyloximino;
C₃-C₆-alkenyl or C₃-C₆-alkynyl, where these groups in turn may carry from one to five halogen atoms;
phenyl which is unsubstituted or monosubstituted to trisubstituted by C₁-C₄-alkyl or C₁-C₄-alkoxy or monosubstituted to pentasubstituted by halogen;
a 5-membered aromatic heterocylic structure which has from one to four nitrogen atoms in the ring, is bonded via a nitrogen atom and may carry from one to three of the following substituents: halogen, nitro, cyano, C₁-C₆-alkyl, C₁-C₆-haloalkyl, C₁-C₆-alkoxy or C₁-C₆-alkylthio; a group -N=CR⁶R⁷, where R⁶ and R⁷ are each C₁-C₂₀-alkyl which may carry phenyl, C₁-C₄-alkoxy and/or C₁-C₄-alkylthio, or are each phenyl or together form a C₃-C₁₂-alkylene chain which may carry from one to three C₁-C₃-alkyl groups;
a radical 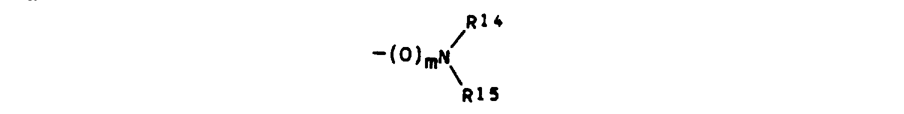 where m is 0 or 1 and R¹⁴ and R¹⁵ may be identical or different and have the following meanings:
hydrogen;
C₁-C₆-alkyl, C₃-C₆-alkenyl or C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C3-C6-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, bis-C₁-C₆-dialkylamino, C₃-C₆-cycloalkyl or phenyl which may carry from one to three of the following substituents: halogen, nitro, cyano, C₁-C₆-alkyl, C₁-C₆-haloalkyl, C₁-C₆-alkoxy or C₁-C₆-alkylthio; C₃-C₆-cycloalkyl which may carry from on to three of the following substituents: halogen, nitro, cyano, C₁-C₆-alkyl, C₁-C₆-haloalkyl, C₁-C₆-alkoxy or C₁-C₆-alkylthio; phenyl which may carry from one to three of the following substituents: halogen, nitro, cyano, C₁-C₆-alkyl, C₁-C₆-haloalkyl, C₁-C₆-alkoxy or C₁-C₆-alkylthio;
R⁶ together with R⁷ form a 4-membered to 7-membered alkylene chain which may carry from one to three of the following substituents: halogen, nitro, cyano, C₁-C₆-alkyl, C₁-C₆-haloalkyl, C₁-C₆-alkoxy or C₁-C₆-alkylthio and in which one CH₂ group may be replaced with oxygen, sulfur or -NH;
a group 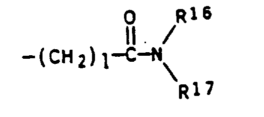 where R¹⁶ and R¹⁷ are each hydrogen, C₁-C₆-alkyl, C₃-C₆-alkenyl or C₃-C₆-alkynyl and 1 is 1, 2, 3 or 4;
or a group
-(CH₂)ₗ-S(=O)ₖ-R¹⁸
where R¹⁸ is C₁-C₆-alkyl, C₃-C₆-alkenyl or C₃-C₆-alkynyl, 1 is 1, 2, 3 or 4 and k is 0, 1 or 2;
a radical OR⁵, where R⁵ is a group 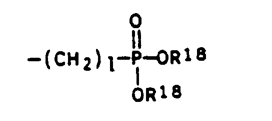 where R¹⁸ and 1 have the abovementioned meanings; or a radical 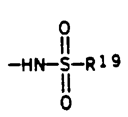 where R¹⁹ is C₁-C₆-alkyl or phenyl, which in turn may carry from one to four of the following substituents: halogen, nitro, cyano or C₁-C₆-alkyl;
R is C₁-C₄-alkyl, C₁-C₄-alkoxy, C₁-C₄-haloalkoxy or halogen;
R³ is hydrogen or, together with R⁴, is a group CH₂=CH-CH=CH₂ or N=CH-CH=CH₂;
R⁴ is hydrogen, halogen, C₁-C₄-alkoxy, C₁-C₄-haloalkoxy, aryloxy, C₁-C₄-haloalkyl, formyl or C₁-C₈-alkoxycarbonyl; a phenyl radical which may be monosubstituted to trisubstituted by halogen, nitro, cyano, C₁-C₆-alkyl, C₁-C₆-haloalkyl, C₁-C₆-alkoxy or C₁-C₆-alkylthio or, where halogen is a substituent, in a phenyl radical which is monosubstituted to pentasubstituted by halogen, nitro, cyano, C₁-C₆-alkyl, C₁-C₆-haloalkyl, C₁-C₆-alkoxy or C₁-C₆-alkylthio 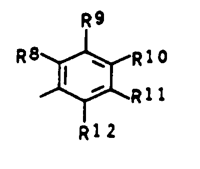 where R⁸, R⁹, R¹⁰, R¹¹ and R¹ are each hydrogen, halogen, cyano or nitro;
C₃-C₆-alkenyl, C₃-C₆-alkenyloxy, C₃-C₆-alkynyloxy or C₃-C₆-alkynyl, where these groups in turn may carry from one to five halogen atoms;
di-C₁-C₄-alkylamino or C₃-C₈-cycloalkyl which may carry from one to three C₁-C₄-alkyl radicals;
C₁-C₁₀-alkoxycarbonyl or C₁-C₄-alkylthio;
phenoxy where the aromatic radical may carry from one to five halogen atoms and/or from one to three of the following radicals: C₁-C₄-alkyl, C₁-C₄-haloalkyl, C₁-C₄-haloalkoxy, C₁-C₄-alkoxy or C₁-C₄-alkylthio;
C₁-C₁₀-alkyl or -alkoxy which may carry from one to five halogen atoms and/or one of the following radicals: C₁-C₄-alkoxy, C₁-C₄-alkylthio, phenyl or phenoxy, where the aromatic radicals in turn may carry from one to five halogen atoms and/or from one to three of the following radicals: C₁-C₄-alkyl, C₁-C₄-haloalkyl, C₁-C₄-haloalkoxy or C₁-C₄-alkylthio;
or R⁴ is a 5-membered heteroaromatic structure having from one to four nitrogen atoms or one or two nitrogen atoms and in addition one sulfur or oxygen atom in the ring, or is a benzofused 5-membered heteroaromatic structure having from one to three nitrogen atoms or one nitrogen atom and in addition one oxygen or sulfur atom in the ring, which heteroaromatic structures may carry from one to three halogen atoms and/or from one to three of the following radicals: nitro, cyano, C₁-C₄-alkyl, C₁-C₄-alkylthio, C₁-C₄-haloalkyl or phenyl which is unsubstituted or substituted by from one to three halogen atoms and/or from one to three methyl groups;
a thienyl or benzothienyl radical which may carry from one to three halogen atoms and/or from one to three of the following radicals: C₁-C₄-alkyl, C₁- or C₂-haloalkyl or nitro;
a naphthyl or quinolyl radical which may carry from one to three of the following substitutents: halogen, nitro, cyano, C₁-C₆-alkyl, C₁-C₆-haloalkyl, C₁-C₆-alkoxyor C₁-C₆-alkylthio;
or oxygen when Y is nitrogen and n is 1;
n is 1 when Y is carbon or when Y is nitrogen and R⁴ is oxygen, and 0 when Y is nitrogen and R⁴ is not oxygen; X is oxygen or sulfur, and
Y is nitrogen when R³ is hydrogen and n is 0 or when R⁴ is oxygen and n is 1; carbon when R³ and R⁴ together form a group CH₂=CH-CH=CH₂ or N=CH-CH=CH₂ and carbon when R³ is hydrogen and n is 1;
and environmentally compatible salts of the compounds I.

2. A salicylaldehyde or salicylic acid derivative of the formula as claimed in claim 1, wherein R is methoxy, X is oxygen and Y and R¹, R³ and R⁴ have the meanings stated in claim 1.

3. A herbicide containing a compound of the formula I as claimed in claim 1 and conventional inert additives.

4. A method for controlling undesirable plant growth, wherein the undesirable plants and/or their habitat is or are treated with a herbicidal amount of a derivative I as claimed in claim 1.

5. An agent for influencing plant growth, containing a salicylaldehyde or salicylic acid derivative of the formula I as claimed in claim 1 and conventional inert additives.

6. A method for regulating plant growth, wherein a bioregulatory amount of a salicylaldehyde or salicylic acid derivative of the formula I as claimed in claim 1 is allowed to act on the seeds, the plants and/or their habitat.

7. A fungicide containing a compound of the formula I as claimed in claim 1 and conventional inert additives.

8. A novel sulfone of the formula III 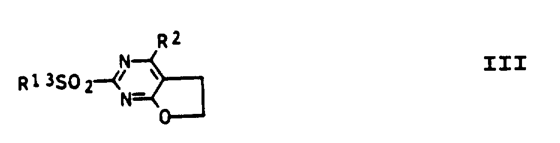 where R is C₁-C₄-alkyl, C₁-C₄-alkoxy, C₁-C₄-haloalkoxy or halogen and R¹³ is C₁-C₁₂-alkyl or is phenyl which is unsubstituted or substituted by alkyl, halogen or alkoxy.

9. A process for the preparation of a compound of the formula I as claimed in claim 1, wherein a corresponding salicylic acid or thiosalicylic acid derivative of the formula II 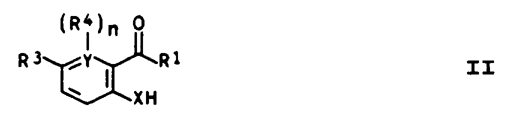 is reacted in a conventional manner with a sulfone of the formula III 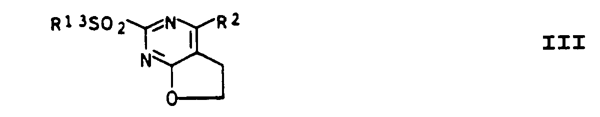 where R¹³SO₂ is a nucleofugic leaving group, in the presence of an inorganic or organic base.

10. A process for the preparation of a sulfone of the formula III as claimed in claim 8, wherein a corresponding 2-alkylthio- or 2-phenylthio-5,6-dihydrofuran[2,3-d]pyrimidine 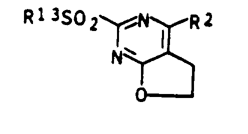 is treated with an oxidizing agent under conventional mild conditions.

## Revendications

1. Dérivés de l'aldéhyde salicylique et de l'acide salicylique et leurs analogues sulfurés, répondant à la formule I, 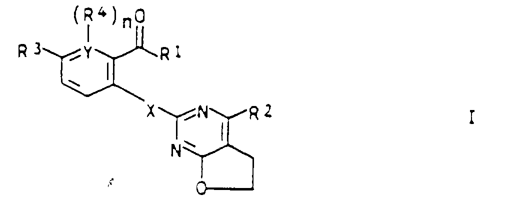 dans laquelle les symboles ont les significations suivantes :
R¹ représente : l'hydrogène,
un groupe succinylimidoxy ;
un radical hétéroaromatique à cinq chaînons contenant un à trois atomes d'azote et qui peut porter un à quatre atomes d'halogènes et/ou un ou deux des substituants suivants : alkyle en C1-C4, halogénoalkyle en C1-C4, alcoxy en C1-C4, halogénoalcoxy en C1-C4 et/ou alkylthio en C1-C4 ;
un groupe -OR⁵ dans lequel
R⁵ représente
l'hydrogène, un cation de métal alcalin, un équivalent d'un cation de métal alcalino-terreux ou un ion organique ;
un groupe cycloalkyle en C3-C12 qui peut porter un à trois substituants alkyle en C1-C4 ;
un groupe alkyle en C1-C10 qui peut porter un à cinq atomes d'halogènes et/ou un des substituants suivants : alcoxy en C1-C4, alkylthio en C1-C4, cyano, (alkyle en C1-C8) carbonyle, (alcoxy en C1-C8) carbonyle, cycloalkyle en C3-C12, phényle, phénoxy ou phénylcarbonyle, les radicaux aromatiques pouvant eux-mêmes porter un à cinq atomes d'halogènes et/ou un à trois des substituants suivants : alkyle en C1-C4, halogénoalkyle en C1-C4, alkoxy en C1-C4, halogénoalcoxy en C1-C4 et/ou alkylthio en C1-C4 ;
un groupe alkyle en C1-C10 qui peut porter un à cinq atomes d'halogènes et porte l'un des substituants suivants : un radical hétéroaromatique à cinq chaînons contenant un à trois atomes d'azote ou un atome d'azote et un atome d'oxygène ou de soufre, qui peut porter un à quatre atomes d'halogène et/ou un ou deux des substituants suivants : alkyle en C1-C4, halogénoalkyle en C1-C4, alcoxy en C1-C4, halogénoalcoxy en C1-C4 et/ou alkylthio en C1-C4 ;
un groupe alkyle en C2-C6 qui porte en position 2 l'un des substituants suivants : alcoxyimino en C1-C6, alcényloxyimino en C3-C6, halogénoalcényloxyimino en C3-C6 ou benzyloxyimino ;
un groupe alcényle en C3-C6 ou alcynyle en C3-C6 qui peut lui-même porter un à cinq atomes d'halogènes ;
un groupe phényle non substitué ou portant un à trois substituants alkyle en C1-C4 ou alcoxy en C1-C4 ou un à cinq substituants halogéno ;
un hétérocycle aromatique à cinq chaînons relié par l'intermédiaire d'un atome d'azote et contenant un à quatre atomes d'azote dans le cycle, qui peut porter un à trois des substituants suivants : halogéno, nitro, cyano, alkyle en C1-C6, halogénoalkyle en C1-C6, alcoxy en C1-C6, alkylthio en C1-C6 ;
un groupe -N=CR⁶R⁷ dans lequel
R⁶ et R⁷ représentent chacun un groupe alkyle en C1-C20 qui peut porter un groupe phényle, un groupe alcoxy en C1-C4 et/ou un groupe alkylthio en C1-C4, un groupe phényle, ou bien R⁶ et R⁷ forment ensemble une chaîne alkylène en C3-C12 qui peut porter un à trois groupes alkyle en C1-C3 ;
un groupe 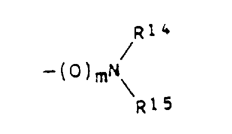 dans lequel m est égal à 0 ou 1 et R¹⁴ et R¹⁵, qui peuvent avoir des significations identiques ou différentes, représentent chacun :
l'hydrogène ;
un groupe alkyle en C1-C6, alcényle en C3-C6, alcymyle en C3-C6, ces groupes pouvant porter chacun un à cinq atomes d'halogènes et/ou un ou deux des substituants suivants : alcoxy en C1-C6, alcényloxy en C3-C6, alcynyloxy en C3-C6, alkylthio en C1-C6, alcénylthio en C3-C6, alcynylthio en C3-C6, halogénoalcoxy en C1-C6, cyano, (alkyle en C1-C6) carbonyle, (alcényle en C3-C6) carbonyle, alcynyle en C3-C6 carbonyle, alcoxy en C1-C6 carbonyle, (alcényloxy en C3-C6) carbonyle, (alcynyloxy en C3-C6) carbonyle, bis-di(alkyle en C1-C6)amino, cycloalkyle en C3-C6, phényle qui peut lui même porter un à trois des substituants suivants : halogéno, nitro, cyano, alkyle en C1-C6, halogénoalkyle en C1-C6, alcoxy en C1-C6, alkylthio en C1-C6 ;
un groupe cycloalkyle en C3-C6, qui peut porter un à trois des substituants suivants : halogéno, nitro, cyano, alkyle en C1-C6, halogénoalkyle en C1-C6, alcoxy en C1-C6, alkylthio en C1-C6 ;
un groupe phényle qui peut porter un à trois des substituants suivants : halogéno, nitro, cyano, alkyle en C1-C6, halogénoalkyle en C1-C6, alcoxy en C1-C6, alkylthio en C1-C6 ;
R⁶ et R⁷ forment ensemble une chaîne alkylène de 4 à 7 chaînons qui peut porter un à trois des substituants suivants : halogéno, nitro, cyano, alkyle en C1-C6, halogénoalkyle en C1-C6, alcoxy en C1-C6, alkylthio en C1-C6, et dans laquelle un groupe CH₂ peut être remplacé par l'oxygène, le soufre ou-NH- ;
un groupe 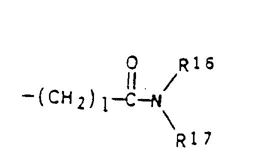 dans lequel R¹⁶ et R¹⁷ représentent chacun l'hydrogène, un groupe alkyle en C1-C6, alcényle en C3-C6 ou alcynyle en C3-C6 et I est un nombre égal à 1, 2, 3 ou 4 ;
ou un groupe
-(CH₂)ₗ-S(=O)ₖ-R¹⁸
dans lequel R¹⁸ représente un groupe alkyle en C1-C6, alcényle en C3-C6 ou alcynyle en C3-C6, I est égal à 1, 2, 3 ou 4 et k est égal à 0, 1 ou 2 ; un groupe OR⁵ dans lequel R⁵ représente un groupe 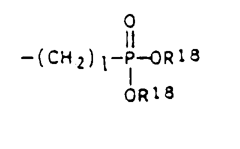 dans lequel R¹⁸ et I ont des significations indiquées ci-dessus ; ou un goupe 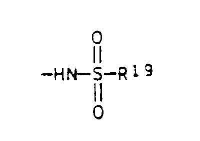 dans lequel R¹⁹ représente un groupe alkyle en C1-C6 ou phényle qui peut lui-même porter un à quatre des substituants suivants : halogéno, nitro, cyano, alkyle en C1-C6;
R représente un groupe alkyle en C1-C4, alcoxy en C1-C4, halogénoalcoxy en C1-C4 ou un halogène ;
R³ représente l'hydrogène ou forme avec R⁴ un groupe CH₂=CH-CH=CH₂ ou N=CH-CH=CH₂ ;
R⁴ représente l'hydrogène, un halogène, un groupe alcoxy en C1-C4, halogénoalcoxy en C1-C4, aryloxy, halogénoalkyle en C1-C4, formyle, (alcoxy en C1-C8) carbonyle ;
un groupe phényle portant éventuellement un à trois des substituants halogéno, nitro, cyano, alkyle en C1-C6, halogénoalkyle en C1-C6, alcoxy en C1-C6, alkylthio en C1-C6, et, dans le cas, d'un substituant halogéno, un à cinq des substituants halogéno, nitro, cyano, alkyle en C1-C6, halogénoalkyle en C1-C6, alcoxy en C1-C6, alkylthio en C1-C6 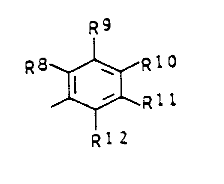
R⁸ et R¹ ayant les significations suivantes :
l'hydrogène, un halogène, un groupe cyano, nitro ;
un groupe alcényle en C3-C6, alcényloxy en C3-C6, alcynyloxy en C3-C6 ou alcynyle en C3-C6, ces groupes pouvant eux-mêmes porter un à cinq atomes d'halogènes ;
un groupe di-(alkyle en C1-C4) amino, cycloalkyle en C3-C8 qui peut porter un à trois groupes alkyle en C1-C4 ;
un groupe (alcoxy en C1-C10) carbonyle, alkylthio en C1-C4 ;
un groupe phénoxy dont le noyau aromatique peut porter un à cinq atomes d'halogènes et/ou un à trois des substituants suivants : alkyle en C1-C4, halogénoalkyle en C1-C4, halogénoalcoxy en C1-C4, alcoxy en C1-C4, alkylthio en C1-C4;
un groupe alkyle ou alcoxy en C1-C10 qui peut porter un à cinq atomes d'halogènes et/ou un des substituants suivants : alcoxy en C1-C4, alkylthio en C1-C4, phényle ou phénoxy, les radicaux aromatiques pouvant eux-mêmes porter un à cinq atomes d'halogènes et/ou un à trois des substituants suivants : alkyle en C1-C4, halogénoalkyle en C1-C4, halogénoalcoxy en C1-C4, alkylthio en C1-C4 ;
un radical hétéroaromatique à cinq chaînons contenant un à quatre atomes d'azote et/ou un à deux-atomes d'azote et un atome de soufre ou d'oxygène dans le cycle ou un radical hétéroaromatique à cinq chaînons condensé avec un noyau benzénique, contenant un à trois atomes d'azote ou un atome d'azote et un atome d'oxygène ou de soufre dans le cycle, qui peut porter un à trois atomes d'halogènes et/ou un à trois des substituants suivants : nitro, cyano, alkyle en C1-C4, alkylthio en C1-C4, halogénoalkyle en C1-C4 ou phényle lui-même non substitué ou portant un à trois atomes d'halogènes et/ou un à trois groupes méthyle ;
un groupe thiényle ou benzothiényle qui peut porter un à trois atomes d'halogènes et/ou un à trois des substituants suivants : alkyle en C1-C4, halogénoalkyle en C1-C2 ou nitro ;
un groupe naphtyle ou quinoléinyle qui peut porter un à trois des substituants suivants : halogéno, nitro, cyano, alkyle en C1-C6, halogénoalkyle en C1-C6, alcoxy en C1-C6, alkylthio en C1-C6 ;
ou l'oxygène lorsque Y représente l'azote et que n est égal à 1 ;
n est égal à 1 lorsque Y représente le carbone ou lorsque Y représente l'azote et R⁴ l'oxygène, et à 0 lorsque Y représente l'azote et que R⁴ a une signification autre que l'oxygène ;
X représente l'oxygène ou le soufre ;
Y représente l'azote lorsque R³ représente l'hydrogène et que n est égal à 0 ou lorsque R⁴ représente l'oxygène et que n est égal à 1 ; le carbone lorsque R³ et R⁴ forment ensemble un groupe CH₂=CH-CH=CH₂ ou N=CH-CH=CH₂ ou le carbone lorsque R³ représente l'hydrogène et que n est égal à 1 ;
et les sels non polluants des composés I.

2. Dérivés de l'aldéhyde salicylique et de l'acide salicylique de formule I de la revendication 1, dans laquelle R représente un groupe méthoxy et X l'oxygène, Y et R¹, R³ et R⁴ ayant les significations indiquées dans la revendication 1.

3. Produit herbicide contenant un composé de formule I de la revendication 1 et des additifs usuels inertes.

4. Procédé pour combattre les croissances de végétaux indésirables, caractérisé en ce que l'on traite les végétaux indésirables et/ou leur habitat par une quantité herbicide efficace d'un dérivé I de la revendication 1.

5. Produit pour agir sur la croissance des végétaux, contenant un dérivé de l'aldéhyde salicylique ou de l'acide salicylique de formule I de la revendication 1 et des additifs inertes usuels.

6. Procédé pour la régulation de la croissance des végétaux, caractérisé en ce que l'on fait agir sur les semences, les végétaux et/ou leur habitat, une quantité biorégulatrice efficace d'un dérivé d'aldéhyde salicylique ou d'acide salicylique de formule générale I de la revendication 1.

7. Produit fongicide contenant un composé de formule I de la revendication 1 et des additifs inertes usuels.

8. Nouvelles sulfones de formule générale III 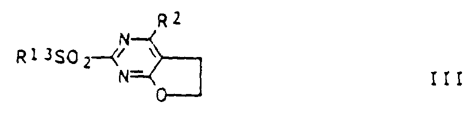 dans laquelle les symboles ont les significations suivantes :
R représente un groupe alkyle en C1-C4, alcoxy en C1-C4, halogénoalcoxy en C1-C4 ou halogène,
R¹³ représente un groupe alkyle en C1-C12, un groupe phényle non substitué ou portant des substituants alkyle, halogéno ou alcoxy.

9. Procédé de préparation des composés de formule I de la revendication 1, caractérisé en ce que l'on fait réagir un dérivé correspondant de l'acide salicylique ou de l'acide thiosalicylique, de formule II 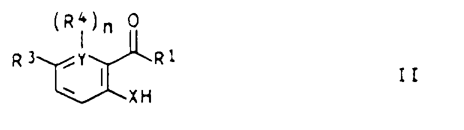 de manière connue en soi, avec une sulfone de formule III 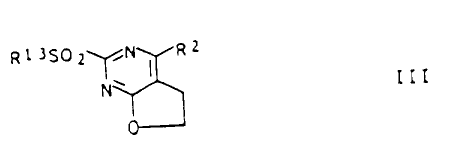 dans laquellel R¹³SO₂ représente un groupe éliminable nucléofuge, en présence d'une base organique ou minérale.

10. Procédé de préparation des sulfones de formule III de la revendication 8, caractérisé en ce que l'on traite par un agent oxydant, dans des conditions ménagées usuelles, une 2-alkylthio- ou 2-phénylthio-5, 6-dihydrofuranne [2, 3-d] pyrimidine correspondante
